# EUROPÄISCHE PATENTSCHRIFT

(11) **EP 2 395 961 B1**
(45) Veröffentlichungstag und Bekanntmachung des Hinweises auf die Patenterteilung: **23.07.2014**
(21) Anmeldenummer: 10707209.2
(22) Anmeldetag: 11.02.2010
(51) Int. Cl.: A61F 13/42, A61M 1/36

(54) **VORRICHTUNG ZUM DETEKTIEREN VON FEUCHTIGKEIT FÜR EINE VORRICHTUNG ZUR ÜBERWACHUNG EINES ZUGANGS ZU EINEM PATIENTEN, INSBESONDERE ZUR ÜBERWACHUNG DES GEFÄSSZUGANGS BEI EINER EXTRAKORPORALEN BLUTBEHANDLUNG**
APPARATUS FOR DETECTING MOISTURE FOR AN APPARATUS FOR MONITORING THE ACCESS TO A PATIENT, IN PARTICULAR FOR MONITORING THE VASCULAR ACCESS DURING EXTRACORPOREAL BLOOD TREATMENT
DISPOSITIF DE DÉTECTION D'HUMIDITÉ DESTINÉ À UN DISPOSITIF DE SURVEILLANCE D'UN POINT D'ACCÈS À UN PATIENT, EN PARTICULIER POUR LA SURVEILLANCE DU POINT D'ACCÈS AUX VAISSEAUX LORS D'UN TRAITEMENT SANGUIN EXTRACORPOREL

(30) Priorität: 14.02.2009 DE 102009008885
(43) Veröffentlichungstag der Anmeldung: 21.12.2011
(73) Patentinhaber: Fresenius Medical Care Deutschland GmbH, 61352 Bad Homburg v.d.H. (DE)
(72) Erfinder: KÖNIG, Christoph, 65207 Wiesbaden/Auringen (DE); SCHRÖRS, Alexander, 60389 Frankfurt (DE); WÜPPER, Andreas, 64572 Büttelborn (DE); HEIDE, Alexander, 65817 Eppstein (DE); KLEWINGHAUS, Jürgen, 61440 Oberursel (DE)
(74) Vertreter: Oppermann, Frank
(86) Internationale Anmeldenummer: PCT/EP2010/000832
(87) Internationale Veröffentlichungsnummer: WO 2010/091852

(56) Entgegenhaltungen:
- US-A- 5 579 765
- US-B1- 6 445 304

## Beschreibung

Die Erfindung bezieht sich auf eine Vorrichtung zum Detektieren von Feuchtigkeit zur Verwendung bei einer Vorrichtung zur Überwachung eines Zugangs zu einem Patienten für eine Einrichtung, mit der über eine Schlauchleitung eine Flüssigkeit zu einem Patienten geführt und/oder von einem Patienten abgeführt wird, insbesondere zur Überwachung des Gefäßzugangs bei einer extrakorporalen Blutbehandlung, bei der Blut eines Patienten über eine arterielle Schlauchleitung, die eine arterielle Punktionskanüle aufweist, von dem Patienten abgeführt wird, und über eine venöse Schlauchleitung, die eine venöse Punktionskanüle aufweist, dem Patienten wieder zugeführt. Darüber hinaus betrifft die Erfindung eine Vorrichtung zur Überwachung eines Zugangs zu einem Patienten, die über eine Vorrichtung zum Detektieren von Feuchtigkeit verfügt. Des Weiteren betrifft die Erfindung eine Blutbehandlungsvorrichtung mit einem extrakorporalen Blutkreislauf, der eine arterielle Schlauchleitung mit einer arteriellen Kanüle und eine venöse Schlauchleitung mit einer venösen Kanüle aufweist, wobei die extrakorporale Blutbehandlungsvorrichtung über eine Vorrichtung zur Überwachung des arteriellen und/oder venösen Gefäßzugangs verfügt.

Auf dem Gebiet der Medizintechnik ist eine Vielzahl von Einrichtungen bekannt, mit denen über eine Schlauchleitung Patienten Flüssigkeiten entnommen oder Flüssigkeiten den Patienten zugeführt werden können. Dabei erfolgt der Zugang zu dem Patienten im Allgemeinen mit einem Katheter zum Einführen in Körperorgane oder einer Kanüle zum Punktieren von Gefäßen. Während der Untersuchung oder Behandlung ist ein ordnungsgemäßer Zugang zu dem Patienten sicherzustellen. Daher ist es erforderlich, den Patientenzugang zu überwachen.

Einen ordnungsgemäßen Zugang zu dem Patienten setzen insbesondere auch die extrakorporalen Blutbehandlungsvorrichtungen voraus, die über einen extrakorporalen Blutkreislauf verfügen. Zu den bekannten extrakorporalen Blutbehandlungsvorrichtungen zählen beispielsweise Dialysevorrichtungen und Zellseparatoren, die einen Zugang zu dem Gefäßsystem des Patienten erforderlich machen. Bei der extrakorporalen Blutbehandlung wird dem Patienten über eine arterielle Schlauchleitung mit einer arteriellen Punktionskanüle Blut entnommen, das dem Patienten über eine venöse Schlauchleitung mit einer venösen Punktionskanüle wieder zugeführt wird.

Trotz regelmäßiger Überwachung des Gefäßzugangs durch das Krankenhauspersonal besteht grundsätzlich die Gefahr, dass die Punktionskanüle unbemerkt aus dem Blutgefäß des Patienten herausrutscht. Während ein Herausrutschen der arteriellen Kanüle mit einem Ansaugen von Luft in die arterielle Schlauchleitung verbunden ist, führt das Herausrutschen der venösen Kanüle zu dem gefürchteten Freifluss des Blutes in die Umgebung. Wenn das Herausrutschen der venösen Kanüle daher nicht sofort erkannt wird, besteht die Gefahr, dass der Patient verblutet. Selbst wenn die venöse Kanüle nicht vollständig herausgerutscht ist, besteht dennoch die Gefahr, dass über den gesamten Behandlungszeitraum relativ große Mengen von Blut an der Punktionsstelle austreten können.

Zur Überwachung des Gefäßzugangs sind verschiedene Vorrichtungen unterschiedlicher Ausbildung bekannt. Die bekannten Überwachungsvorrichtungen greifen im Allgemeinen auf die standardmäßig in den Blutbehandlungsvorrichtungen vorhandenen Sicherheitsvorrichtungen zurück, die bei einem nicht ordnungsgemäßen Gefäßzugang eine sofortige Unterbrechung des extrakorporalen Blutkreislaufs auslösen.

Es sind Vorrichtungen zur Überwachung eines Gefäßzugangs bekannt, die über eine Vorrichtung zum Detektieren von Feuchtigkeit verzügen, um an der Punktionsstelle das Austreten von Blut erkennen zu können.

Die bekannten Vorrichtungen zum Detektieren von Feuchtigkeit zur Verwendung bei den bekannten Überwachungsvorrichtungen für einen Patientenzugang sind als auf die Wunde oder Punktionsstelle aufzulegendes Pad ausgebildet. Das Pad besteht aus einem saugfähigen Material, in den ein Feuchtigkeitssensor eingebettet ist. Nachteilig ist, dass Fehlalarme beispielsweise auf Grund von Schweiß auf der Haut oder anderer Körperflüssigkeiten möglich sind, da mit dem Feuchtigkeitssensor nicht unterschieden werden kann, ob die Feuchtigkeit an der Wund- oder Punktionsstelle auf eine Blutung oder beispielsweise Schweißbildung zurückzuführen ist.

Eine Vorrichtung zur Überwachung eines Patientenzugangs, die über eine als Pad ausgebildete Vorrichtung zum Detektieren von Feuchtigkeit verfügt, ist beispielsweise aus der WO 2006/008866 A1 bekannt. Die bekannte Vorrichtung zum Detektieren von Feuchtigkeit ist als Matte ausgebildet, die unter den Arm des Patienten gelegt wird, der für den Zugang zum Gefäßsystem punktiert wird. Die Matte setzt sich aus mehreren Lagen unterschiedlicher Materialien zusammen. Zwischen den oberen und unteren Schichten ist der Feuchtigkeitssensor eingebettet, der aus einer Materiallage besteht, die mit einer meanderförmigen Leiterbahn bedruckt ist, deren Enden als Kontakte ausgebildet sind. Die Feuchtigkeit wird dadurch erkannt, dass sich der Widerstand zwischen den Kontakten der Leiterbahn ändert. Dem Problem von Fehlalarmen auf Grund von Schweiß versucht die bekannte Vorrichtung dadurch zu begegnen, dass die Lage mit der Leiterbahn von einem hydrophoben Filter mit Poren bedeckt ist. Die Anzahl und Größe der Poren soll dafür sorgen, dass nur größere, aber nicht geringere Mengen an Feuchtigkeit an den Feuchtigkeitssensor gelangen.

Die US 2005/0038325 A1 beschreibt eine Vorrichtung zum Detektieren von Feuchtigkeit für eine Vorrichtung zur Überwachung eines Patientenzugangs, welche als Pad ausgebildet ist, das auf die Punktionsstelle aufgelegt wird. Das Pad hat wieder einen mehrschichtigen Aufbau, wobei der Feuchtigkeitssensor wieder zwischen den oberen und unteren Schichten eingebettet ist. Die bekannte Vorrichtung zum Detektieren von Feuchtigkeit zeichnet sich dadurch aus, dass an der Punktionsstelle austretendes Blut an der auf der Haut des Patienten aufliegenden Unterseite des Pads in die untere Schicht eintritt. Über den Abstand zwischen dem Feuchtigkeitssensor und der unteren Schicht soll die Sensitivität derart eingestellt werden, dass Fehlalarme auf Grund von Schweiß vermieden werden.

Das aus der US 2005/0038325 A1 bekannte Pad weist einen Abschnitt auf, an dem die Kanüle befestigt ist. Dieser Abschnitt ist über eine Sollbruchlinie von dem Abschnitt getrennt, in dem der Feuchtigkeitssensor angeordnet ist. Dadurch wird erreicht, dass bei einem unbeabsichtigten Zug an der Schlauchleitung, die zum Rausrutschen der Kanüle führt, nur der Abschnitt des Pads abgerissen wird, an dem die Kanüle befestigt ist, und der Abschnitt mit dem Feuchtigkeitssensor aber an der Punktionsstelle verbleibt. Dadurch ist sichergestellt, dass auch bei unbeabsichtigtem Zug an der Schlauchleitung Alarm ausgelöst wird.

Eine als Pad ausgebildete Vorrichtung zum Detektieren von Feuchtigkeit, bei der das Blut an der Unterseite des Pads eintritt, ist auch aus der US 6,445,304 B1 bekannt. Mit einer ausreichenden Dicke des blutdurchlässigen porösen Trägermaterials für den Feuchtigkeitssensor und mit einem geeigneten Abstand der Leiterbahnen des Feuchtigkeitssensors soll vermieden werden, dass ein nur gelegentliches Aussickern von Blut oder geringe Mengen von anderen Körperflüssigkeiten einen Fehlalarm auslösen.

Aus der US 6,445,304 B1 ist weiterhin bekannt, in einem auf die Punktionsstelle aufzulegenden Pad eine kreisförmige Ausnehmung vorzusehen, von der sich ein Schlitz bis an den Rand des Pads erstreckt. Diese besondere Ausbildung des Pads ermöglicht es, den Pad auch dann anzulegen, wenn der Gefäßzugang des Patienten bereits mit einer Kanüle punktiert ist. Der Schlitz erlaubt es, den Pad von der Seite über die bereits gelegte Kanüle zu schieben, die sich dann durch die kreisförmige Ausnehmung erstreckt. Wenn das Pad vor dem Anlegen der Kanüle auf die Haut des Patienten aufgelegt wird, erweist sich der Schlitz aber als nachteilig, da das geschlitzte Pad an Stabilität verliert.

Die US 5,579,765 A beschreibt eine Vorrichtung zur Erkennung des Austretens von Blut an einer Punktionsstelle, die zwei auf die Haut des Patienten aufzulegende Bandagen umfasst, von denen die eine Bandage einen Feuchtigkeitssensor und die andere Bandage eine Alarmeinheit aufnimmt. Der Feuchtigkeitssensor umfasst elektrische Leiter, die in ein für Blut saugfähiges Material eingebettet sind. Das Auftreten einer Blutung wird durch die Veränderung des elektrischen Widerstandes zwischen den Leitern erkannt, die mit dem saugfähigen Material in Kontakt stehen, das von Blut durchtränkt ist.

Aus der US 6,445,304 B1 ist eine Vorrichtung zur Erkennung von Blutungen bekannt, die auf dem gleichen Funktionsprinzip beruht. Die elektrische Leiterschleife ist in ein saugfähiges Material eingebettet, das auf die Haut des Patienten aufgelegt wird.

Der Erfindung liegt die Aufgabe zu Grunde, eine einfach zu handhabende Vorrichtung zum Detektieren von Feuchtigkeit zur Verwendung bei einer Vorrichtung zur Überwachung eines Zugangs zu einem Patienten zu schaffen, bei der das Risiko des Auftretens von Fehlalarmen beispielweise auf Grund von Schweiß verringert ist. Darüber hinaus liegt der Erfindung die Aufgabe zu Grunde, eine Vorrichtung zur Überwachung eines Zugangs zu einem Patienten, insbesondere zur Überwachung des Gefäßzugangs bei einer extrakorporalen Blutbehandlung zu schaffen, die sich einfach handhaben lässt und eine sichere Erkennung des Austretens von Blut an der Punktionsstelle erlaubt. Eine Aufgabe der Erfindung ist auch, eine extrakorporale Blutbehandlungsvorrichtung mit einer derartigen Vorrichtung zur Überwachung des Gefäßzugangs anzugeben.

Die Lösung dieser Aufgaben erfolgt erfindungsgemäß mit den Merkmalen der Patentansprüche 1, 13 und 14. Vorteilhafte Ausführungsformen der Erfindung sind Gegenstand der Unteransprüche.

Die erfindungsgemäße Vorrichtung zum Detektieren von Feuchtigkeit ist als eine auf die Haut des Patienten aufzulegende Abdeckung aus einem flexiblen Material mit einer der Haut des Patienten zugewandten Unterseite und einer der Haut des Patienten abgewandten Oberseite ausgebildet. Die Abdeckung (Pad) weist eine Ausnehmung auf, durch die eine Kanüle für den Patientenzugang geführt werden kann, und einen Feuchtigkeitssensor mit Kontaktelementen zum Anschluss desselben auf. Bei dem Feuchtigkeitssensor kann es sich um einen Einmalartikel oder um einen mehrfach verwendbaren Sensor handeln.

Die erfindungsgemäße Vorrichtung zeichnet sich dadurch aus, dass die Abdeckung an der Unterseite für Flüssigkeit nicht durchlässig ist. Im Gegensatz zu den bekannten Vorrichtungen zum Detektieren von Feuchtigkeit ist es daher erforderlich, dass die Flüssigkeit, beispielsweise das Blut an der Punktionsstelle nicht an der Unterseite in die Abdeckung eindringt, sondern auf die Oberseite der Abdeckung gelangt, um von dem Feuchtigkeitssensor detektiert werden zu können. Dadurch wird vermieden, dass nur geringe Mengen von Flüssigkeiten, beispielsweise Schweiß, von der Unterseite in die Abdeckung eindringen und zu Fehlalarmen führen können.

Die erfindungsgemäße Vorrichtung kann nicht nur bei Blutbehandlungsvorrichtungen verwendet werden, die einen Gefäßzugang über eine Kanüle oder Nadel schaffen, sondern ist prinzipiell auch zur Verwendung bei Port-Systemen und Kathetern geeignet.

Bei einer bevorzugten Ausführungsform der erfindungsgemäßen Vorrichtung ist die an der Unterseite für Flüssigkeit undurchlässige Abdeckung an der Unterseite aber für Luft und Wasserdampf durchlässig. Dadurch wird der Tragekomfort der erfindungsgemäßen Abdeckung verbessert.

Wenn der Feuchtigkeitssensor nicht in die Abdeckung eingebettet ist, sondern auf der Oberseite der Abdeckung angeordnet ist, braucht die Abdeckung an der Oberseite nicht für Flüssigkeit durchlässig zu sein. Bei einer bevorzugten Ausführungsform ist die Abdeckung an der Oberseite aber für Flüssigkeit durchlässig.
Die Abdeckung kann als ein Pad mit zwei Längsseiten und zwei Schmalseiten ausgebildet sein. Das Pad braucht nicht im Wesentlichen rechteckförmig zu sein, sondern kann auch im Wesentlichen rund oder oval sein.

Die Ausnehmung in der Abdeckung für den Durchtritt von Blut ist vorzugsweise eine im Wesentlichen kreisförmige oder ovale Ausnehmung. Es sind aber auch alle anderen Formen möglich. Beispielsweise kann die Ausnehmung auch im Wesentlichen quadratisch oder reckteckförmig sein.

Die Ausnehmung in der Abdeckung erlaubt, die Kanüle durch die Ausnehmung zu führen. Dabei kann die Kanüle vor oder nach dem Anbringen der Abdeckung gelegt werden. Es ist aber auch möglich, die Kanüle unter der Abdeckung anzuordnen. Dann kann die Kanüle mit der Abdeckung an der Haut des Patienten befestigt werden.

Für den Fall, dass die Kanüle durch die Ausnehmung in der Abdeckung geführt werden soll, sieht eine besonders bevorzugte Ausführungsform vor, dass die Abdeckung eine Sollbruchlinie aufweist, die sich von der Ausnehmung in der Abdeckung bis zum Rand der Abdeckung erstreckt. Für den Fall, dass die erfindungsgemäße Detektionsvorrichtung vor dem Anlegen der Kanüle auf die Haut des Patienten aufgelegt wird, hat die Abdeckung eine ausreichende Stabilität. Die Kanüle kann dann durch die Ausnehmung der Abdeckung in die Haut des Patienten gestochen werden. Die erfindungsgemäße Vorrichtung erlaubt dem Anwender aber auch in gewohnter Weise zuerst die Kanüle zu legen, um dann die Abdeckung auf die Haut des Patienten aufzulegen. Wenn der Anwender an seinem gewohnten Arbeitsablauf festhalten will, braucht er die Abdeckung nur an der Sollbruchlinie einzureißen, so dass ein Schlitz zum seitlichen Einführen der Kanüle in die Abdeckung bis in die Ausnehmung entsteht. Daher kann die erfindungsgemäße Vorrichtung flexibel, aber auch einfach gehandhabt werden.

Die Abdeckung kann unterschiedliche Größen haben. Sie sollte einerseits eine ausreichende Größe haben, um die Punktionsstelle vollständig abzudecken, andererseits sollte sie auch nicht so groß sein, dass die Punktion behindert wird. Vorzugsweise hat die vorzugsweise rechteckförmige Abdeckung eine Länge von 8 bis 12 Zentimetern und eine Breite von 3 bis 5 Zentimetern. Sie kann auch eine oder mehrere Laschen oder Einbuchtungen aufweisen.

Da sich die Ausnehmung für den Durchtritt von Blut bzw. zur Durchführung der Kanüle in der Abdeckung befindet, wird die Punktionsstelle von der Abdeckung von allen Seiten umschlossen. Die Ausnehmung sollte eine ausreichende Größe haben, so dass der Durchtritt von Blut und die Durchführung der Kanüle leicht möglich ist. Wenn die Ausnehmung eine ausreichende Größe hat, ist sichergestellt, dass kleinere Sickerblutungen nicht sofort zu einem Alarm führen können, da diese kleinen Blutmengen dann nicht an die Oberfläche der Abdeckung gelangen könnten. Eine ausreichend große Ausnehmung erlaubt dem Anwender aber auch, die Kanüle mit Klebestreifen durch die Ausnehmung hindurch zu fixieren. Der Durchmesser der vorzugsweise kreisförmigen Ausnehmung sollte zwischen 20 - 35 mm liegen.

Eine weitere bevorzugte Ausführungsform sieht vor, dass die Abdeckung ein flexibles Trägermaterial aufweist, das ein oder mehrere Schichten oder Lagen umfassen kann. Auf der Oberseite des ein- oder mehrschichtigen Trägermaterials sind mindestens eine Leiterbahn aus einem flexiblen Material und mindestens zwei Kontaktelemente zum Anschluss der mindestens einen Leiterbahn aufgebracht, um den eigentlichen Feuchtigkeitssensor zu bilden.

Das Trägermaterial für den Feuchtigkeitssensor ist vorzugsweise ein medizinisches Vlies. Aufgrund der offenen Struktur des Vliesstoffes ist das Trägermaterial sowohl für Luft- als auch Wasserdampf durchlässig, wodurch sich die Abdeckung angenehm auf der Haut tragen lässt. Der Vliesstoff ist biokompatibel, hautverträglich, weich und flexibel und kann beispielsweise aus einem Cellulose-/Polyester-Fasergemisch bestehen. Aufgrund des flexiblen Vliesstoffs kann sich die Abdeckung jeder Shunt-Geometrie anpassen und auf jede Punktionsstelle geklebt werden. Die bevorzugte Dicke des Vliesstoffes liegt zwischen 50 und 500 µm, vorzugsweise 100 - 200 µm, insbesondere 150 µm. Der Vliesstoff sollte keine großen Poren oder sonstige Löcher aufweisen. Der maximale Durchmesser der Poren oder Löcher sollte kleiner als 0,5 mm sein, so dass die auf dem Trägermaterial aufgebrachten Leiterbahnen nicht unterbrochen werden. Damit die Leiterbahnen nicht auseinander gerissen werden können, sollte der Vliesstoff nur relativ schwer dehnbar sein. In Längs- und Querrichtung sollte sich der Vliesstoff auch bei den in der Praxis maximal anzunehmenden Zugkräften nicht mehr als 20 % dehnen lassen.

Bei einer besonders bevorzugten Ausführungsform sind die Leiterbahnen auf das Trägermaterial aufgedruckt. Für das Bedrucken des Trägermaterials mit den Leiterbahnen sind dem Fachmann geeignete Druckverfahren bekannt. Vorzugsweise werden die Leiterbahnen im Siebdruck auf das Trägermaterial gedruckt, da der Siebdruck das Aufbringen relativ großer Schichtdicken erlaubt, die erforderlich sind, um auf den relativ rauen, flexiblen und nur etwas dehnbaren Vliesstoff mechanisch stabile und robuste Leiterbahnen aufbringen zu können.

Zur Schaffung der Leiterbahnen wird auf den Vliesstoff im Siebdruck vorzugsweise eine Paste auf Silberbasis, gebunden in einem thermoplastischen Harz aufgebracht, das beim Aushärten unter Wärmeeinfluss evaporiert. Die Schichtdicke ist auf der Oberfläche des Vliesstoffes nicht gleichmäßig, da die Paste mehr oder weniger tief in die nicht homogene Oberfläche des Vliessstoffes einsickert. Die Schichtdicke der Leiterbahnen liegt vorzugsweise zwischen 10 µm und 150 µm.

Eine besonders bevorzugte Ausführungsform des Feuchtigkeitssensors sieht eine erste zu einem Abschlusswiderstand führende Leiterbahn und eine zweite von dem Abschlusswiderstand abgehende zweite Leiterbahn vor, wobei die Enden der ersten und zweiten Leiterbahn mit den Kontaktelementen elektrisch verbunden sind, die vorzugsweise auf das Trägermaterial aufgedruckt sind. Auch der Abschlusswiderstand ist vorzugsweise auf das Trägermaterial aufgedruckt. Die Paste für den gedruckten Widerstand besteht vorzugsweise aus einer Mischung aus Kohlenstoffpartikeln und isolierenden Partikeln, beide eingebunden in ein thermoplastisches Harz, das ebenfalls beim Aushärten unter Wärmeeinfluss evaporiert.

Die beiden als Elektroden ausgebildeten Leiterbahnen haben einen nur geringen elektrischen Widerstand, der bei ca. 1 Ω/cm liegt, während der Abschlusswiderstand hingegen einen großen Widerstand hat, der im zwei- oder mehrstelligen KΩ-Bereich liegen kann. Wenn die Leiterbahnen durch einen Flüssigkeitstropfen miteinander verbunden werden, fällt der Wert des zwischen den Kontaktelementen gemessenen Widerstands weit unter den Wert des Widerstands des Abschlusswiderstandes. Aufgrund dieser Widerstandsänderung kann das Auftreten von Feuchtigkeit detektiert werden. Über die Form der Leiterbahnen und den Abstand zwischen den beiden Leiterbahnen kann eingestellt werden, ab welcher Tropfengröße der Sensor ansprechen soll.

Anstelle eines Feuchtigkeitssensors, der seinen Widerstand in Abhängigkeit von der Feuchtigkeit ändert, kann auch ein dem Fachmann bekannter Feuchtigkeitssensor in die Abdeckung integriert werden, der seine Kapazität in Abhängigkeit von der Feuchtigkeit ändert.

Die mindestens eine Leiterbahn auf dem Trägermaterial kann einen unterschiedlichen Verlauf haben. Vorzugsweise bildet die mindestens eine Leiterbahn eine die Ausnehmung in der Abdeckung zumindest teilweise umschließende Leiterschleife. Die Leiterschleife sollte so geformt sein, dass sie beim Aufreißen der Sollbruchlinie nicht einreißt. Dabei sollte die Leiterschleife die Ausnehmung in der Abdeckung möglichst von allen Seiten eng umschließen.

Bei der besonders bevorzugten Ausführungsform, die von gedruckten Leiterbahnen Gebrauch macht, muss das vorzugsweise aus Vliesstoff bestehende Trägermaterial Eigenschaften aufweisen, die eine Bedruckbarkeit erlauben. So darf die Oberfläche des Vliesstoffes beispielsweise bei einer Druckfarbe auf Wasserbasis nicht hydrophob sein. Auch muss der Vliesstoff der für die Trocknung der Druckpaste oder -farbe nötigen Belastung, beispielsweise der Wärme oder UV-Strahlung, standhalten können.

Mit der Druckpaste oder -farbe für die Leiterbahnen oder Kontaktelemente können auch weitere Beschriftungen und Markierungen auf die Abdeckung aufgedruckt werden, die dem Anwender spezielle Hinweise auf die Handhabung geben können.

Bei einer weiteren besonders bevorzugten Ausführungsform weist die Abdeckung eine die Abdeckung in zwei Teilabschnitte aufteilende Sollbruchlinie auf, wobei der Feuchtigkeitssensor in einem der beiden Teilabschnitte der Abdeckung angeordnet ist. An dem anderen Teil der Abdeckung kann die Punktionskanüle befestigt werden. Für den Fall, dass die Punktionskanüle herausgerissen werden sollte, wird nur der Teilabschnitt, an dem die Kanüle befestigt ist, von der Abdeckung abgerissen. Dadurch ist sichergestellt, dass der andere Teilabschnitt, an dem sich der Feuchtigkeitssensor befindet, an der Haut des Patienten verbleibt.

Der Teilabschnitt der Abdeckung, an dem die Kanüle befestigt wird, kann unterschiedlich ausgebildet sein. Er kann einen Teilbereich der Abdeckung bilden, d.h. von dem anderen Teilabschnitt seitlich umschlossen sein, oder als ein nach außen abstehender Streifen oder eine Lasche ausgebildet sein. Allein entscheidend ist, dass sich dieser Teilabschnitt leicht von dem anderen Teilabschnitt lösen kann, wenn der Teilabschnitt für die Befestigung der Kanüle auf Zug beansprucht wird.

Eine weitere besonders bevorzugte Ausführungsform sieht eine zu einem zweiten Abschlusswiderstand führende dritte Leiterbahn und eine von dem zweiten Abschlusswiderstand abgehende vierte Leiterbahn vor, wobei die Enden der dritten und vierten Leiterbahn mit den Kontaktelementen elektrisch verbunden sind, die auf das Trägermaterial aufgebracht sind. Dadurch wird eine Parallelschaltung von zwei Widerständen geschaffen. Bei dieser Ausführungsform ist es möglich, die zweite Sollbruchlinie, die den ersten von dem zweiten Teilabschnitt der Abdeckung trennt, derart anzuordnen, dass sie entweder durch die erste und/oder zweite Leiterbahn oder durch die dritte und/oder vierte Leiterbahn verläuft. Dadurch wird erreicht, dass beim Abreißen der Nadel nur die erste und/oder zweite Leiterbahn oder die dritte und/oder vierte Leiterbahn durchtrennt wird, was zu einer entsprechenden Änderung des Gesamtwiderstandes der Parallelschaltung der beiden Abschlusswiderstände führt. Ein Abreißen der Kanüle führt daher nicht zu einer vollständigen Zerstörung des Feuchtigkeitssensors. Wenn die Änderung des Widerstands überwacht wird, kann sofort erkannt werden, dass die Kanüle abgerissen ist. Aus dem Betrag der Widerstandsänderung kann unterschieden werden, ob die Kanüle abgerissen ist oder eine Blutung aufgetreten ist.

Bei einer weiteren bevorzugten Ausführungsform ist die Abdeckung als Klebepflaster ausgebildet, das an seiner Unterseite eine Klebeschicht aufweist. Diese Klebschicht kann von einer Abdeckschicht, beispielsweise eine Folie oder einem Papier, abgedeckt sein, das vor dem Aufbringen der Abdeckung auf die Haut des Patienten abgezogen wird. Die Klebeschicht erstreckt sich vorzugsweise nur über einen Teilabschnitt der Unterseite der Abdeckung, so dass der andere Teilabschnitt der Abdeckung an der Unterseite frei von der Klebeschicht ist. Dieser von der Klebeschicht freie Teil der Abdeckung ist für die Anbringung der Kontaktelemente des Feuchtigkeitssensors bestimmt. Da der Teilabschnitt mit den Kontaktelementen des Feuchtigkeitssensors nicht an der Haut des Patienten haftet, kann ein geeigneter Stecker selbst dann leicht an der Abdeckung befestigt werden, wenn der Teilabschnitt mit der Klebeschicht bereits an der Haut des Patienten haftet. Dadurch wird die Handhabung weiter vereinfacht. Auch kann ein Teil der Abdeckung frei von der Klebeschicht sein, der eine Lasche bilden soll, an der sich die Abdeckschicht, beispielsweise die Folie oder das Papier, von dem Trägermaterial leicht abziehen lässt.

Der nicht mit der Klebeschicht versehene Teilabschnitt der Abdeckung erlaubt auch später ein einfaches Abziehen der Abdeckung von der Haut des Patienten, da der von der Klebeschicht freie Teilabschnitt eine Lasche bildet, die leicht mit den Fingern zu fassen ist.

Die Klebeschicht an der Unterseite des Sensors kann mit geeigneten Beschichtungs- oder Druckverfahren direkt oder indirekt (Transferdruck) aufgebracht werden. Der Kleber sollte hautverträglich und biokompatibel sein. Er kann beispielsweise aus einem Copolymerisat (z.B. Acrylat) bestehen und wasserlöslich sein. Anstelle eines Acrylat-Klebers können aber auch andere dem Fachmann bekannte Kleber verwendet werden. Die Klebekraft des Sensors kann über die Menge an aufgebrachten Kleber eingestellt werden. Die Schichtdicke der Klebeschicht hängt dabei von dem verwendeten Kleber, dem verwendeten Vlies und der gewünschten Klebekraft ab und sollte für jede Werkstoffpaarung individuell ermittelt werden. Der Klebstoff sollte so ausgewählt und die Klebekraft so eingestellt werden, dass sich die Abdeckung relativ schmerzfrei wieder ablösen lässt. Bedingt durch die große Klebfläche des Sensors kann ein sicherer Halt der Abdeckung auch mit einer relativ geringen Klebkraft gewährleistet werden.

Die auf die Unterseite der Abdeckung befindliche Klebeschicht ist vorzugsweise eine für Flüssigkeit undurchlässige Klebeschicht, die aber vorzugsweise für Dampf durchlässig ist. Daher kann für das Trägermaterial der Abdeckung ein für Flüssigkeit durchlässiges Material, insbesondere ein Vlies verwendet werden.

Die erfindungsgemäße Vorrichtung zum Detektieren von Feuchtigkeit wird vorzugsweise einzeln und steril verpackt bereitgestellt. Als Sterilisationsverfahren kommen sowohl eine Ethylenoxid-Sterilisation (EO), als auch alle Arten der Strahlensterilisation in Frage. Bevorzugt wird die γ- Sterilisation.

Die erfindungsgemäße Vorrichtung zur Überwachung eines Zugangs zu einem Patienten, insbesondere zur Überwachung des Gefäßzugangs bei einer extrakorporalen Blutbehandlung verfügt neben der erfindungsgemäßen Vorrichtung zum Detektieren von Feuchtigkeit über eine Auswerteinheit, an die der Feuchtigkeitssensor der Vorrichtung zum Detektieren von Feuchtigkeit angeschlossen werden kann. Die Übertragung der Daten zwischen der Vorrichtung zum Detektieren von Feuchtigkeit und der Auswerteinheit der Überwachungsvorrichtung erfolgt vorzugsweise über ein Verbindungskabel. Das Verbindungskabel weist vorzugsweise einen Anschlussteil mit federnden Kontakten auf, die mit den Kontaktelementen des Feuchtigkeitssensors der Vorrichtung zum Detektieren von Feuchtigkeit verbunden werden können.

Die Auswerteinheit weist eine Einheit zum Bestimmen des Widerstandes und/oder der Kapazität des Feuchtigkeitssensors der Vorrichtung zum Detektieren von Feuchtigkeit auf. Vorzugsweise ist die Auswerteinheit derart ausgebildet, dass sowohl resistive Feuchtigkeitssensoren, die ihren Widerstand in Abhängigkeit von der Feuchtigkeit ändern, als auch kapazitive Feuchtigkeitssensoren, die ihre Kapazität in Abhängigkeit von der Feuchtigkeit ändern, ausgewertet werden können.

Es ist bekannt, für die resistive Messung eine Gleich- oder Wechselspannung an den resistiven Feuchtigkeitssensor zu legen, die zu einem Stromfluss im Sensor in Abhängigkeit von der an dem Sensor anliegenden Feuchtigkeit führt. Um sowohl einen resistiven, als auch kapazitiven Feuchtigkeitssensor vermessen zu können, werden an den Feuchtigkeitssensor als Messsignal vorzugsweise Gleichspannungspulse, vorzugsweise von ca. 25 µs Dauer, mit einem Tastverhältnis vorzugsweise ≤ 50% gelegt. Dabei stellt das Messsignal für die Dauer von ca. 25 µs eine Gleichspannung für den resistiven Sensor zur Verfügung, aber auch eine Wechselspannung für den kapazitiven Sensor in einer Frequenz von ca. 40 KHz. Ein weiterer Vorteil der Messung mit kurzen Gleichspannungsimpulsen ist, dass die Gleichspannungsimpulse keine Gefahr für den Patienten darstellen.

Für die resistive bzw. kapazitive Messung wird das Rechtecksignal vorzugsweise über einen resistiven bzw. kapazitiven Spannungsteiler an den Feuchtigkeitssensor angelegt. Verändert sich der Widerstand bzw. die Kapazität des Feuchtigkeitssensors in Abhängigkeit von der Feuchtigkeit, ändert sich auch das Spannungsteilerverhältnis und damit der zu ermittelnde Widerstand bzw. die zu ermittelnde Kapazität. Für die Auswertung eines resistiven Feuchtigkeitssensor wird die Spannung am Spannungsteiler nur während des Gleichspannungsimpulses gemessen.

Da das Verbindungskabel zwischen dem Feuchtigkeitssensor und der Auswerteinheit neben dem Ohmschen Anteil auch einen kapazitiven Anteil hat, ist es möglich, durch eine geeignete Messung zu überprüfen, ob mit dem Verbindungskabel eine elektrische Verbindung zwischen dem Feuchtigkeitssensor und der Auswerteinheit der Überwachungsvorrichtung hergestellt ist, d.h. das Verbindungskabel richtig an die Vorrichtung zum Detektieren von Feuchtigkeit und die Überwachungsvorrichtung angeschlossen ist.

Bei einer besonders bevorzugten Ausführungsform der Überwachungsvorrichtung werden die Messergebnisse von der Auswerteinheit drahtlos an die Einrichtung übertragen, mit der über eine Schlauchleitung Flüssigkeit dem Patienten zugeführt und/oder von dem Patienten abgeführt wird, insbesondere an die extrakorporale Blutbehandlungsvorrichtung. Hierzu verfügt die Überwachungsvorrichtung über eine Sendeeinheit und eine Empfangseinheit als räumlich getrennte Einheiten. Die Empfangseinheit ist dabei vorzugsweise Bestandteil der extrakorporalen Blutbehandlungsvorrichtung. Es ist aber auch möglich, die Auswerteinheit mit der Steuereinheit der extrakorporalen Blutbehandlungsvorrichtung über ein weiteres Verbindungskabel zu verbinden. Eine bidirektionale Datenübertragung ist dann möglich, wenn zwei Sende-/Empfangseinheiten als räumlich getrennte Einheiten vorgesehen sind. Beispielsweise kann eine Sende-/Empfangseinheit der Überwachungsvorrichtung mit einer Sende-/Empfangseinheit kommunizieren, die in der Blutbehandlungsvorrichtung vorgesehen ist.

Die Empfangseinheit oder Sende-/Empfangseinheit ist vorzugsweise ein Teil der zentralen Steuereinheit der extrakorporalen Blutbehandlungsvorrichtung oder an die Steuereinheit angeschlossen, so dass für den Fall des Austritts von Blut an der Punktionsstelle die Steuereinheit geeignete Eingriffe in die Maschinensteuerung vornehmen kann, beispielsweise der Blutfluss unterbrochen und Alarm gegeben werden kann.

Die Überwachungsvorrichtung wirkt vorzugsweise mit der Steuereinheit der Blutbehandlungsvorrichtung derart zusammen, dass die Blutbehandlungsvorrichtung für den Fall einer Störung in einen für den Patienten sicheren Zustand überführt wird. Hierzu kann die Steuereinheit die im extrakorporalen Blutkreislauf angeordnete Blutpumpe stoppen und/oder die arterielle und/oder venöse Schlauchklemme im extrakorporalen Blutkreislauf schließen. Auch kann die Steuereinheit einen akustischen und/oder optischen Alarm geben.

Zur drahtlosen Übertragung der Messergebnisse stehen dem Fachmann die bekannten Funkmodule mit Frequenzen von beispielsweise 2.4 GHz sowie geeignete Kommunikationsprotokolle zur Verfügung. Die Messergebnisse können in zyklischen Abständen an die Dialysemaschine übertragen werden.

Zum Anschluss des Verbindungskabels an die Kontaktelemente des Feuchtigkeitssensors verfügt das Verbindungskabel über einen Anschlussteil mit federnden Kontakten, die elektrisch mit den Kontaktelementen des Feuchtigkeitssensors verbunden werden können. Dabei ist es möglich, an dem Feuchtigkeitssensor ein oder mehrere Kontaktpaare vorzusehen, um den Stecker an unterschiedlichen Seiten leicht an den Feuchtigkeitssensor anschließen zu können. Beispielsweise ist es möglich, ein Kontaktpaar quer zur Längsrichtung der Abdeckung und ein Kontaktpaar in Längsrichtung der Abdeckung vorzusehen. Dadurch wird die Handhabung weiter vereinfacht.

Die Stromversorgung der Auswerteinheit der Überwachungsvorrichtung erfolgt vorzugsweise mit einer Batterie oder einem Akku. Die Auswerteinheit kann von dem Anwender eingeschaltet werden, wodurch automatisch eine Zuordnung zwischen Auswerteinheit und Dialysemaschine erfolgen kann. Hierzu verfügt die Überwachungsvorrichtung vorzugsweise über einen Taster oder Schalter. Die Auswerteinheit kann vorzugsweise vom Anwender aber mit dem Taster oder Schalter nicht wieder ausgeschaltet werden, so dass sichergestellt ist, dass die Überwachungsvorrichtung immer aktiv ist. Ein Ausschalten der Überwachungsvorrichtung sollte nur über die Dialysemaschine möglich sein, wenn sichergestellt ist, dass die Behandlung unterbrochen werden soll oder beendet ist. Ein entsprechendes Signal zum Ausschalten der Überwachungsvorrichtung kann von der Dialysemaschine beispielsweise über Funk an die Auswerteinheit der Überwachungsvorrichtung übermittelt werden.
Um einen möglichst langen Betrieb der Überwachungsvorrichtung sicherstellen zu können, sollte der Stromverbrauch möglichst gering sein. Dies kann dadurch erreicht werden, dass die Sende- und Empfangseinheit der Überwachungsvorrichtung nur während der Datenübertragung aktiv ist. Auch sollte die Auswerteinheit nur dann aktiv sein, wenn die Messwerte von dem Feuchtigkeitssensor ausgelesen werden. Nach der Erfassung und Auswertung der Messsignale wird die Überwachungsvorrichtung vorzugsweise in einen Stromspar-/Schlafmodus versetzt. Mit diesen Maßnahmen lässt sich der Stromverbrauch verringern und somit die Betriebsdauer der Überwachungsvorrichtung erheblich verlängern, ohne die Batterie austauschen oder den Akku wieder laden zu müssen.

Die erfindungsgemäße Vorrichtung zur Überwachung eines Zugangs zu einem Patienten kann eine separate Einheit bilden oder auch Bestandteil der Einrichtung sein, mit der dem Patienten eine Flüssigkeit zugeführt und/oder von dem Patienten Flüssigkeit abgeführt wird, insbesondere Bestandteil der extrakorporalen Blutbehandlungsvorrichtung sein. Wenn die erfindungsgemäße Überwachungsvorrichtung Bestandteil der Blutbehandlungsvorrichtung ist, kann die erfindungsgemäße Überwachungsvorrichtung von bestimmten Baugruppen oder Bauteilen Gebrauch machen, die in der Blutbehandlungsvorrichtung ohnehin vorhanden sind.

Prinzipiell sind auch noch andere Ausführungsformen denkbar, z. B. dass das Pad die Punktionsstelle komplett abdeckt und dabei sogar über mehrere Ausnehmungen, z. B. mehrere Öffnungen verfügt, mit dem Zweck, dass die zu detektierende Flüssigkeit durch die Öffnungen auf die äußere Oberfläche des Pads dringen kann und dort die Leiterbahnen benetzt. Es wäre aber auch denkbar, dass das Pad gar keine Ausnehmung aufweist und die Punktionsstelle komplett abdeckt. Bei solchen Ausführungsformen wäre die Punktionsstelle allerdings verdeckt und nicht sichtbar.

Im Folgenden werden verschiedene Ausführungsbeispiele der Erfindung unter Bezugnahme auf die Zeichnungen näher erläutert.

Es zeigen:
- Fig. 1: die wesentlichen Komponenten einer Hämodialysevorrichtung, die über eine Vorrichtung zur Überwachung des arteriellen und venösen Gefäßzugangs verfügt,
- Fig. 2: ein Ausführungsbeispiel der Vorrichtung zum Detektieren von Feuchtigkeit der Vorrichtung zur Überwachung des arteriellen und venösen Gefäßzugangs in der Draufsicht,
- Fig. 3: die Vorrichtung von Fig. 2 in der Unteransicht,
- Fig. 4: einen Schnitt durch die Vorrichtung von Fig. 2,
- Fig. 5: die auf der Haut des Patienten aufliegende Vorrichtung zum Detektieren von Feuchtigkeit von Fig. 2 zusammen mit der Kanüle,
- Fig. 6: das Gehäuse der Vorrichtung zur Überwachung des arteriellen und venösen Gefäßzugangs,
- Fig. 7A und 7B: die Stecker-Einheit des Verbindungskabels zum Anschluss der erfindungsgemäßen Vorrichtung zum Detektieren von Feuchtigkeit,
- Fig. 8A und 8B: ein erstes Ausführungsbeispiel des Anschlussteils des Verbindungskabels,
- Fig. 9A und 9B: ein zweites Ausführungsbeispiel des Anschlussteils des Verbindungskabels,
- Fig. 10A, 10B, 10C: weitere Ausführungsbeispiele der erfindungsgemäßen Vorrichtung zum Detektieren von Feuchtigkeit zusammen mit einer Punktionskanüle,
- Fig. 11: ein weiteres Ausführungsbeispiel der Vorrichtung zum Detektieren von Feuchtigkeit in der Draufsicht,
- Fig. 12: die Vorrichtung von Fig. 11 in der Unteransicht,
- Fig. 13: die Vorrichtung zum Detektieren von Feuchtigkeit von Fig. 12 zusammen mit der Kanüle,
- Fig. 14: ein weiteres Ausführungsbeispiel der Vorrichtung zum Detektieren von Feuchtigkeit in der Draufsicht,
- Fig. 15: die Vorrichtung von Fig. 15 in der Unteransicht,
- Fig. 16: die Vorrichtung zum Detektieren von Feuchtigkeit von Fig. 14 zusammen mit der Kanüle,
- Fig. 17A, 17B, 17C: die Kontaktelemente und der Anschlussteil der Vorrichtung von Fig. 11 oder Fig. 14 in unterschiedlichen Positionen,
- Fig. 18: ein weiteres Ausführungsbeispiel der Vorrichtung zum Detektieren von Feuchtigkeit in der Draufsicht,
- Fig. 19: ein elektrisches Ersatzschaltbild der Vorrichtung von Fig. 18,
- Fig. 20: ein weiteres Ausführungsbeispiel des Gehäuses der Vorrichtung zur Überwachung des arteriellen und venösen Gefäßzugangs mit zwei Stecker-Einheiten,
- Fig. 21 A, 21B: eine Schutzkappe für eine freie Stecker-Einheit,
- Fig. 22: ein weiteres Ausführungsbeispiel der Detektionsvorrichtung und
- Fig. 23: ein weiteres Ausführungsbeispiel der Detektionsvorrichtung.

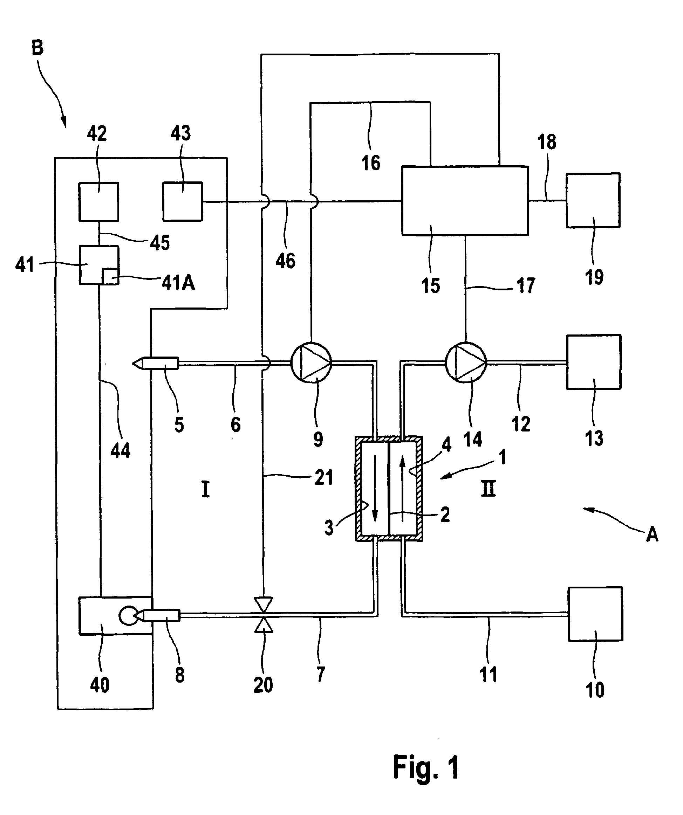

Fig. 1 zeigt die wesentlichen Komponenten einer Hämodialysevorrichtung (A), die über eine Vorrichtung (B) zur Überwachung des arteriellen und venösen Gefäßzugangs verfügt. Bei dem vorliegenden Ausführungsbeispiel ist die Überwachungsvorrichtung (B) Bestandteil der Hämodialysevorrichtung (A). Zunächst wird die Hämodialysevorrichtung unter Bezugnahme auf Fig. 1 beschrieben.

Die Hämodialysevorrichtung weist einen Dialysator 1 auf, der durch eine semipermeable Membran 2 in eine Blutkammer 3 und eine Dialysierflüssigkeitskammer 4 unterteilt ist. An einer Arterie des Patienten ist mittels einer arteriellen Punktionskanüle 5 eine arterielle Schlauchleitung 6 angeschlossen, die zu dem Einlass der Blutkammer 3 des Dialysators führt. Von dem Auslass der Blutkammer 3 des Dialysators geht eine venöse Schlauchleitung 7 ab, die mittels einer venösen Punktionskanüle 8 an einer Vene des Patienten angeschlossen ist. Die arterielle Schlauchleitung 6 ist in eine okkludierende Blutpumpe 9 eingelegt, die das Blut im extrakorporalen Blutkreislauf I fördert.

Der Dialysierflüssigkeitskreislauf II der Hämodialysevorrichtung umfasst eine Dialysierflüssigkeitsquelle 10, an der eine Dialysierflüssigkeitszufuhrleitung 11 angeschlossen ist, die zu dem Einlass der Dialysierflüssigkeitskammer 4 des Dialysators führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet.

Die Steuerung der Dialysevorrichtung übernimmt eine zentrale Steuereinheit 15, die über Steuerleitungen 16, 17 die Blut- und Dialysierflüssigkeitspumpe 9, 14 ansteuert. Die zentrale Steuereinheit 15 ist über eine Datenleitung 18 mit einer Alarmeinheit 19 verbunden, die bei einem Störfall einen optischen und/oder akustischen Alarm gibt.

Stromab der Blutkammer 3 des Dialysators 1 befindet sich an der venösen Schlauchleitung 7 eine elektromagnetisch betätigbare Schlauchklemme 20, die über eine weitere Steuerleitung 21 von der zentralen Steuereinheit 15 geschossen wird, wenn die venöse Punktionskanüle (Nadel) aus dem Gefäßzugang herausrutschen sollte. Darüber hinaus stoppt die Steuereinheit 15 nach dem Herausrutschen der Kanüle die Blutpumpe 9.

Die Überwachungsvorrichtung (B) dient bei dem vorliegenden Ausführungsbeispiel zur Überwachung des venösen Gefäßzugangs. Die Überwachungsvorrichtung (B) verfügt über eine Vorrichtung 40 zur Detektion von Feuchtigkeit, die an der Punktionsstelle angeordnet wird. Diese Detektionsvorrichtung 40 ist in Fig. 1 nur schematisch dargestellt. Darüber hinaus verfügt die Überwachungsvorrichtung über eine Auswerteinheit 41, eine Sendeeinheit 42 und eine Empfangseinheit 43. Anstelle einer Sendeeinheit 42 und einer Empfangseinheit 43 können für eine bidirektionale Datenübertragung aber auch zwei miteinander kommunizierende Sende-/Empfangseinheiten vorgesehen sein. Dabei kann eine der Sende-/Empfangseinheiten auch der Dialysemaschine zugeordnet sein. Die Auswerteinheit 41 der Überwachungsvorrichtung B ist über eine Verbindungsleitung 44 mit der Detektionsvorrichtung 40 elektrisch verbunden. Über eine Datenleitung 45 ist die Auswerteinheit 41 mit der Sendeeinheit 42, während die Empfangseinheit 43 der Überwachungsvorrichtung B über eine Datenleitung 46 mit der zentralen Steuereinheit 15 der Dialysevorrichtung verbunden ist.

Während die Auswerteinheit 41 und Sendeeinheit 42 zusammen mit der Detektionsvorrichtung 40 dem Patienten zugeordnet werden, wird die Empfangseinheit 43 der Dialysevorrichtung zugeordnet. Für den Fall, dass an der Punktionsstelle Blut austritt, erzeugt die Auswerteinheit 41 ein Steuersignal, das die Sendeeinheit 42 aussendet und die Empfangseinheit 43 empfängt. Das Steuersignal wird über die Datenleitung 46 an die zentrale Steuereinheit 15 übertragen, die einen Eingriff in die Blutbehandlung vornimmt. Die zentrale Steuereinheit 15 erzeugt ein Alarmsignal, so dass die Alarmeinheit 19 einen akustischen und/oder optischen Alarm gibt. Darüber hinaus stoppt die Steuereinheit 15 die Blutpumpe 9 und schließt die Schlauchklemme 20.

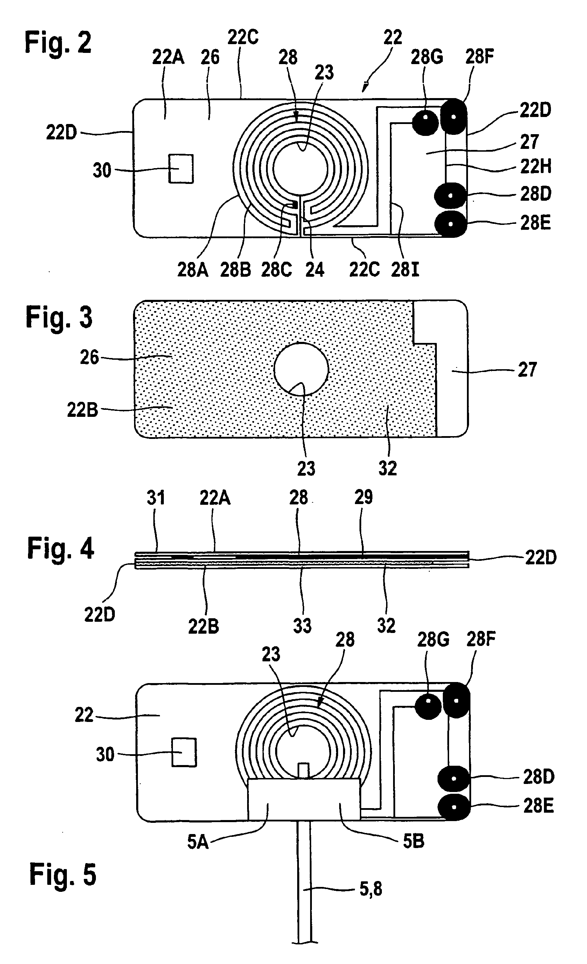

Nachfolgend wird ein erstes Ausführungsbeispiel der auf die Haut des Patienten an der Punktionsstelle aufzulegenden Vorrichtung 40 zum Detektieren von Feuchtigkeit unter Bezugnahme auf die Figuren 2 bis 5 beschrieben. Fig. 2 zeigt die Detektionsvorrichtung 40 in der Draufsicht, während Fig. 3 die Vorrichtung in der Unteransicht zeigt. Fig. 4 zeigt einen Schnitt durch die Vorrichtung. Fig. 5 zeigt die auf der Haut des Patienten aufliegende Vorrichtung in der Draufsicht zusammen mit der Kanüle.

Die Detektionsvorrichtung ist als eine auf die Haut des Patienten aufzulegende Abdeckung 22 aus einem flexiblen Material ausgebildet. Die Abdeckung 22 weist eine der Haut des Patienten abgewandte Oberseite 22A (Fig. 2) und eine auf die Haut des Patienten aufzulegende Unterseite 22B (Fig. 3) auf. Es handelt sich bei der Abdeckung um ein rechteckförmiges Pad mit abgerundeten Ecken, das zwei Längsseiten 22C und zwei Schmalseiten 22D aufweist. Im Zentrum des Pads zwischen den Längs- und Schmalseiten 22C, 22D befindet sich eine kreisförmige Ausnehmung 23. Von der kreisförmigen Ausnehmung 23 erstreckt sich eine Sollbruchlinie 24 in Form einer Perforation bis zu einer der beiden Längsseiten 22C des Pads. Das Pad 22 kann gedanklich in einen ersten breiten Teilabschnitt 26 und einen zweiten schmalen Teilabschnitt 27 unterteilt werden. In dem Pad 22 befindet sich ein Feuchtigkeitssensor 28 zum Detektieren von Feuchtigkeit an der Punktionsstelle. Das Pad 22 besteht aus mehreren Schichten, die nachfolgend beschrieben werden.

Das Pad 22 weist ein biokompatibles, hautverträgliches, weiches Trägermaterial 29 aus einem flexiblen Vliesstoff, beispielsweise aus einem Cellulose-/Polyester-Fasergemisch mit einer Schichtdicke von ca. 150 µm auf. Aufgrund seiner offenen Struktur ist der Vliesstoff sowohl luft- als auch wasserdampfdurchlässig. Der Feuchtigkeitssensor 28 befindet sich auf der Oberseite des Trägermaterials 29. Der Sensor 28 weist zwei Leiterbahnen 28A, 28B auf, von denen die eine Leiterbahn 28A zu einem Abschlusswiderstand 28C führt und die zweite Leiterbahn 28B von dem Abschlusswiderstand abgeht. Die Enden der beiden Leiterbahnen sind mit elektrischen Kontaktelementen 28D und 28E verbunden. Die Leiterbahnen 28A, 28B bilden eine Leiterschleife um die kreisförmige Ausnehmung 23. Dabei liegt die Leiterschleife in dem breiten Teilabschnitt 26 des Pad, während die Kontaktelemente 28D, 28E in dem schmalen Teilabschnitt 27 des Pad liegen.

Die beiden Kontaktelemente 28D und 28E zum Kontaktieren des Feuchtigkeitssensors 28 liegen in dem schmalen Teilabschnitt 27 des Pad 22 nebeneinander in einer Richtung quer zur Längsrichtung des Pad. In dem schmalen Teilabschnitt 27 befinden sich zwei weitere Kontaktelemente 28F und 28G, die elektrisch über weitere Leiterbahnen 28H und 28I mit den anderen Kontaktelementen 28D und 28E elektrisch verbunden sind. Diese Kontaktelemente 28F und 28G sind nebeneinander in Längsrichtung auf dem Pad angeordnet. Beide Kontaktelementpaare ermöglichen den Anschluss des Feuchtigkeitssensors an die Auswerteinheit 23 über das Verbindungskabel 26 mit einem geeigneten Anschlussteil, der nachfolgend noch beschrieben wird. Da zwei Kontaktelementepaare zur Verfügung stehen, kann der Stecker entweder an das eine oder andere Kontaktelementepaar angeschlossen werden. Es ist daher möglich, den Stecker entweder an der Schmal- oder Längsseite mit dem Pad zu verbinden.

Die Leiterbahnen 28A, 28B sowie 28H, 28I sind im Siebdruckverfahren auf die Oberseite des Trägermaterials 29 aufgedruckt. Ebenfalls sind die Kontakte 28D und 28E sowie 28F und 28G auf die Oberseite des Trägermaterials aufgedruckt. Auch der Abschlusswiderstand 28C ist ein gedruckter Widerstand, der auf die Oberseite des Trägermaterials aufgedruckt ist. Neben den Leiterbahnen und Kontaktelementen können noch weitere Markierungen 30 oder dergleichen auf die Oberseite des Trägermaterials 29 aufgedruckt werden, die dem Anwender Hinweise für die Handhabung des Pad geben, beispielsweise zum Anschluss des Steckers an das Pad geben.

Auf der Oberseite des Trägermaterials 29 bzw. der Leiterbahnen und Kontaktelemente befindet sich vorzugsweise eine feuchtigkeitsdurchlässige Deckschicht 31. Damit können Fehlalarme durch unbeabsichtigtes Berühren der Leiterbahnen oder Kontaktelemente vermieden werden. Die Deckschicht 31 erleichtert auch das Säubern des Pad, wenn es mit Flüssigkeiten in Berührung gekommen sein sollte.

An der Unterseite ist das Trägermaterial 29 mit einer für Flüssigkeit undurchlässigen , aber für Dampf durchlässigen Klebeschicht 32 versehen. Die Klebeschicht 32 erstreckt sich aber nur über den breiten Teilabschnitt 26 mit der kreisförmigen Ausnehmung, nicht jedoch über den schmalen Teilabschnitt 27, an dem die Kontaktelemente 28E, 28D und 28F, 28G angeordnet sind. Da der schmale Teilabschnitt 27 mit den Kontaktelementen nicht an der Haut des Patienten haftet, lässt sich der Anschlussteil für den Anschluss des Feuchtigkeitssensors 28 leicht mit dem Pad verbinden. Hierzu kann der schmale Teilabschnitt 27 mit den Fingern angehoben werden. Auch bildet der schmale Teilabschnitt 27 eine Lasche zum späteren Abziehen des Pad von der Haut des Patienten. Die Klebeschicht 32 und der von der Klebeschicht freie Abschnitt der Unterseite des Trägermaterials 29 sind mit einem Silikonpapier 33 kaschiert, das biokompatibel und hautverträglich ist. Es sollte sich leicht von der Klebeschicht 32 lösen lassen, ohne jedoch selbst die Kleberschicht vom Trägermaterial 29 zu lösen. Da das Silikonpapier 33 wegen der fehlenden Klebeschicht nicht an dem schmalen Teilabschnitt 27 des Trägermaterials 29 haftet, kann das Silkonpapier 33 leicht vor dem Auflegen des Pad auf die Haut des Patienten abgezogen werden. Um das Abziehen des Silikonpapiers 33 auch mit Gummihandschuhen zu erleichtern, können auch andere Abschnitte von der Klebeschicht ausgespart sein, beispielsweise an den Längsseiten des Sensors, so dass sich das Silikonpapier von zwei Seiten her abziehen lässt. Auch können an dem Silikonpapier 33 weitere Laschen ausgebildet sein, an denen sich das Papier greifen lässt. Diese Laschen können sich auch am Trägermaterial 29 befinden.

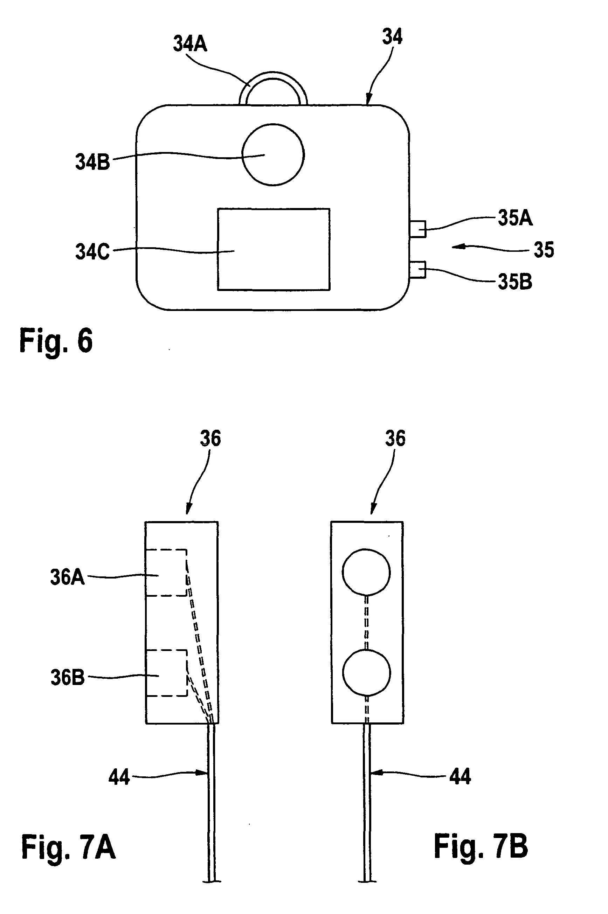

Fig. 6 zeigt in schematischer Darstellung das patientenseitig anzubringende Gehäuse 34, das die Auswerteinheit 41 und Sende- und Empfangseinheit 42 der erfindungsgemäßen Überwachungsvorrichtung B aufnimmt. An der Oberseite weist das Gehäuse 34 einen Bügel 34A auf, in den ein nicht dargestellter Clip eingehängt werden kann, der in der patientennahen Umgebung, beispielsweise am Bett oder an der Kleidung des Patienten angebracht werden kann. Der nicht dargestellte Clip ist nicht Bestandteil der Überwachungsvorrichtung B. Somit kann der Clip, der lösbar am Bügel 34A des Gehäuses 34 zu befestigen ist, bei Beschädigung oder Verschmutzung einfach weggeworfen werden. An dem Gehäuse 34 befindet sich ein Taster 34B zum Einschalten der Auswerteeinheit 41 und der Sende- und Empfangseinheit 42. Mit dem Taster 34B kann das Gerät aber nicht ausgeschaltet werden. Weiterhin ist ein verschließbares Batteriefach 34C zum Einlegen der Batterie für die Stromversorgung vorgesehen. An der Seite des Gehäuses 34 befindet sich eine Stecker-Einheit 35 zum Anschluss der Detektionsvorrichtung 40 an die Auswerteinheit 41 der Überwachungsvorrichtung B. Während die Stecker-Einheit 35 an dem Gehäuse 34 zwei nebeneinander angeordnete Stecker 35A, 35B aufweist, verfügt die Verbindungsleitung 44 an einem Ende über eine entsprechende Buchsen-Einheit 36 mit zwei in gleichem Abstand nebeneinander liegend angeordneten Buchsen 36A, 36B (Fig. 7A und 7B). Die Stecker 35A, 35B und Buchsen 36A, 36B der Stecker- und Buchsen-Einheit 35, 36 sind in der Art von Druckknöpfen ausgebildet, die sich leicht miteinander verbinden und leicht voneinander lösen kann. Die Druckknopf Kontakte sind in der Stecker- und Buchsen-Einheit 35, 36 eingeschlossen, so dass die Kontakte nach dem Verbinden von Stecker und Buchse gegen Reinigungs- und Desinfektionsmittel sowie weitere Verunreinigungen weitgehend resistent sind. Die Stecker- und Buchsen-Einheit 35, 36 erlauben eine dauerhafte Verbindung, die sich leicht lösen lässt.

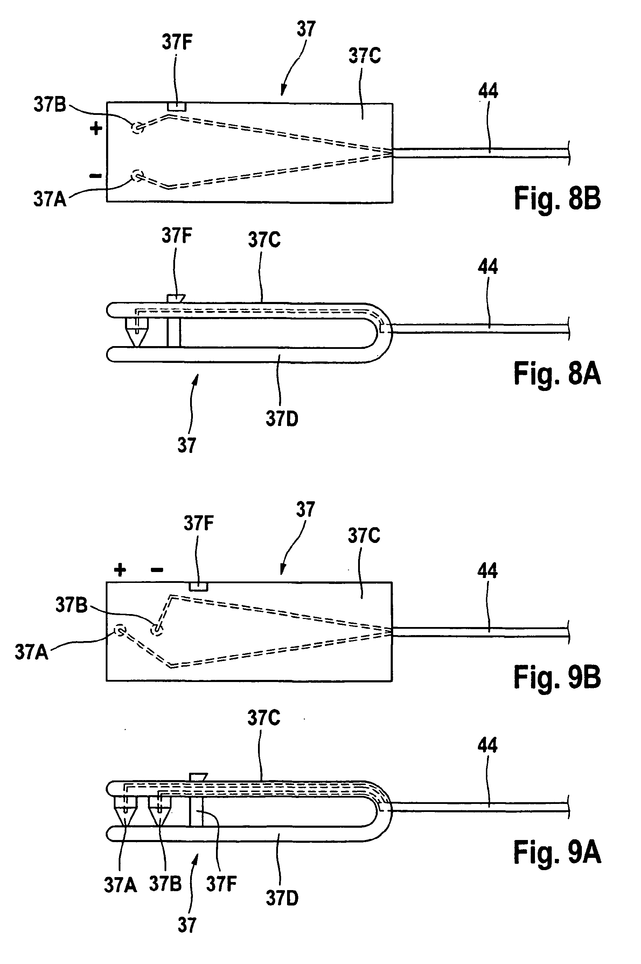

Zum Anschluss des Verbindungskabels 44 an den Feuchtigkeitssensor 28 befindet sich an dem anderen Ende des Verbindungskabels 44 ein Anschlussteil 37. Die Fig. 8A und 8B zeigen eine vereinfachte schematische Darstellung einer ersten Ausführungsform des Anschlussteils 37 in der Seitenansicht und Draufsicht. Mit diesem Anschlussteil 37 kann das Verbindungskabel an die Kontaktelemente 28D, 28E angeschlossen werden, die an der Schmalseite 22D des Pad 22 angeordnet sind. Der Anschlussteil 37 weist zwei stiftförmige Kontakte 37A, 37B auf, die in gleichem Abstand wie die Kontaktelemente 28D, 28E angeordnet sind. Der Anschlussteil 37 ist in der Art einer Klemme ausgebildet, die einen oberen und unteren federnden Bügel 37C, 37D aufweist. Er ist vorzugsweise ein einstückiges Kunststoffteil, wobei der obere und untere Bügel (Schenkel) 37C, 37D beispielsweise über ein Filmscharnier oder dergleichen miteinander federnd verbunden werden können.

Der obere Bügel 37C wird von einem an der Oberseite des oberen Bügels 37C angreifenden Rastelement 37F, das an dem unteren Bügel 37D befestigt ist, in einer Position gehalten, in der die stiftförmigen Kontakte 37A, 37B an dem oberen Bügel 37C entgegen der Federspannung beider Bügel 37C, 37D gegen den unteren Bügel 37C gedrückt werden. Nach Lösen des Rastelements 37F springt der obere Bügel 37C auf, so dass sich der Anschlussteil 37 an das Pad 22 anschließen lässt.

Um eine elektrische Verbindung zwischen den Kontakten 37A, 37B des Anschlussteils 37 und den Kontaktelementen 38D, 38E des Pad herstellen zu können, wird der Anschlussteil 37 bei geöffneten Bügeln auf das Pad seitlich aufgeschoben. Dann werden die beiden Bügel 37C, 37D mit zwei Fingern zusammengedrückt, bis das Rastelement 37F einrastet. Dabei greifen die stiftförmigen Kontaktelemente 37A, 37B des Anschlussteils 37 in den relativ weichen Vliesstoff des Pad, wodurch der Anschlussteil 37 an dem Pad sicher gehalten wird. Um die Kontakte 28E, 28D des Pad 22 besser treffen zu können, können an dem Pad Markierungen oder dergleichen vorgesehen sein, die wie die Kontaktelemente auf das Trägermaterial aufgedruckt sind.

Die Fig. 9A und 9B zeigen eine alternative Ausführungsform des Anschlussteils 37, das sich von dem unter Bezugnahme auf die Fig. 8A und 8B beschriebenen Anschlussteil nur dadurch unterscheidet, dass die beiden Kontakte 37A, 37B nicht nebeneinander, sondern hintereinander angeordnet sind. Daher sind die einander entsprechenden Teile des Anschlussteils 37 auch mit den gleichen Bezugszeichen versehen. Der Anschlussteil 37 von den Fig. 9A und 9B ist zum Anschluss an die beiden Kontaktelemente 28G, 28F bestimmt, die an der Längsseite 22C des Pads 22 angeordnet sind. Es ist aber auch möglich, den Anschlussteil von den Fig. 8A und 8B an den Kontaktelementen 28F, 28G an der Längsseite 22C und den Anschlussteil von den Fig. 9A und 9B an den Kontaktelementen 28D, 28E an der Schmalseite des Pad 22 anzuschließen. Daher ist ein Anschluss an unterschiedlichen Seiten des Pad möglich, wobei der jeweilige Anschlussteil entweder an der Längsseite oder Schmalseite an das Pad angesetzt wird und dann in unterschiedliche Richtungen zeigt.

Die unter Bezugnahme auf die Figuren 2 bis 4 beschriebene Überwachungsvorrichtung B wird in Verbindung mit der extrakorporalen Blutbehandlungsvorrichtung A wird wie folgt gehandhabt.

Fig. 5 zeigt die auf der Haut des Patienten aufliegende Vorrichtung zum Detektieren von Feuchtigkeit zusammen mit der einen Punktionsflügel aufweisenden Kanüle. Für den Fall, dass die arterielle oder venöse Kanüle 5, 8 bereits angeschlossen sind, wird das Pad 22 an der Schwächungslinie 24 (Perforation) eingerissen, so dass ein Schlitz entsteht. Daraufhin wird das Pad 22 über die bereits gelegte Kanüle aufgeschoben. Anschließend kann der an der Kanüle 5, 8 vorgesehene Punktionsflügel 5A, 5B befestig werden, wobei die Punktionsstelle innerhalb der kreisförmigen Ausnehmung 23 des Pad 22 liegt. Wenn nun Blut an der Punktionsstelle austritt, kann das Blut nicht an der für Flüssigkeit undurchlässigen Unterseite in das Pad 22 eindringen. Das Blut kann aber durch die kreisförmige Ausnehmung 23 strömen und auf die Oberseite des Pad 22 gelangen, an dem das Pad für Flüssigkeit durchlässig ist. Das Blut sickert in den Vliesstoff 29 und gelangt an den Feuchtigkeitssensor 28, dessen Widerstand oder Kapazität sich ändert. Die Auswerteinheit 41 misst den Widerstand oder die Kapazität des Feuchtigkeitssensors 28 und vergleicht den Widerstand oder die Kapazität mit einem vorgegebenen Grenzwert. Wenn der Grenzwert überschritten wird, erzeugt die Auswerteinheit 41 ein Steuer- oder Alarmsignal, das die patientenseitige Sende- und Empfangseinheit 42 an die dialysemaschinenseitige Sende- und Empfangseinheit 43 sendet. Die Empfangseinheit 43 erzeugt daraufhin ein Steuersignal, das die zentrale Steuereinheit 15 der Dialysemaschine empfängt. Daraufhin unterbricht die Steuereinheit 15 den Blutfluss durch die venöse Schlauchleitung 7 durch Schließen der elektrisch betätigbaren Schlauchklemme 20, und die Alarmeinheit 19 gibt einen akustischen und/oder optischen Alarm.

Für den Fall, dass die Kanüle 5 oder 8 noch nicht gelegt sein sollte, braucht das Pad 22 an der Schwächungslinie 24 (Perforation) nicht durchtrennt zu werden. In diesem Fall kann die Punktion der Hautstelle nach dem Auflegen des Pad 22 auf die Haut des Patienten durch die kreisförmige Ausnehmung 23 des Pad erfolgen.

Der Abschlusswiderstand 28C des Feuchtigkeitssensors 28 erlaubt sowohl die Überprüfung der Integrität der beiden Leiterbahnen des Feuchtigkeitssensors 28 als auch den richtigen Anschluss des Verbindungskabels 44. Hierfür verfügt die Auswerteinheit 41 der Überwachungsvorrichtung B über eine Einheit, die überprüft, ob der Widerstand des Feuchtigkeitssensors bei einer vorgegebenen Luftfeuchtigkeit oberhalb bzw. unterhalb eines Grenzwertes liegt. Wenn der Widerstand zwischen den Kontaktelementen des Feuchtigkeitssensors stark von dem Abschlusswiderstand abweicht, beispielsweise wesentlich größer als der Abschlusswiderstand ist, wird darauf geschlossen, dass eine Leiterbahn beschädigt oder unterbrochen oder das Verbindungskabel nicht richtig angeschlossen ist. Wenn dies der Fall ist, fordert die Auswerteinheit den Anwender durch ein optisches und/oder akustisches Signal auf, den Feuchtigkeitssensor bzw. den Anschluss der Verbindungsleitung zu überprüfen.

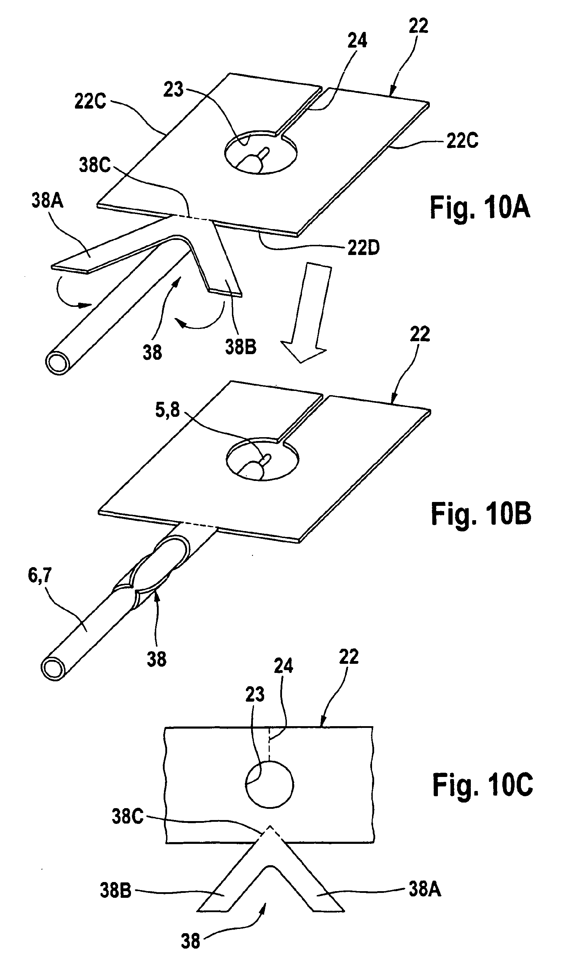

Die Fig. 10A und 10B zeigen eine weitere Ausfiihrungsform des erfindungsgemäßen Pad 22 zusammen mit der Kanüle 5, 8, wobei wieder für die einander entsprechenden Teile die gleichen Bezugszeichen verwandt werden.

Das Pad von den Fig. 10A und 10B weist wieder eine kreisförmige Ausnehmung 23 und eine Schwächungslinie 24 auf. Das Pad von den Fig. 10A und 10B unterscheidet sich von den zuvor beschriebenen Ausführungsformen dadurch, dass an eine Schmalseite 22D eine Sicherungseinrichtung 38 zur Fixierung der Kanüle 5, 8 angeformt ist, mit der sich die zu der Kanüle 5, 8 führende Schlauchleitung 6, 7 fixieren lässt. Bei der Sicherungseinrichtung 38 handelt es sich um einen V-förmigen Streifen, der an der Unterseite mit einer Klebeschicht versehen ist (Pflaster). Das V-förmige Pflaster 38 ist mit dem vorderen Teil an die Schmalseite 22D des Pad 22 angeformt, an den sich die beiden Schenkel 38A, 38B des V-förmigen Pflasters treffen. Dabei ist die Verbindungsstelle 38C zwischen dem Pflaster 38 und dem Pad 22 mit einer Schwächungslinie 38C (Perforation) versehen, die parallel zu der Schmalseite 22D des Pad 22 verläuft. Das V-förmige Pflaster 38 kann aber auch an die Längsseite 22 C des Pad angeformt sein.

Fig. 10A zeigt das Pad 22 vor der Fixierung der Kanüle 6, 7. Zur Befestigung der Kanüle 6, 7 werden die beiden Schenkel 38A, 38B des Pflasters 38 um die zu der Kanüle führende Schlauchleitung 6, 7 fest geschlungen (Fig. 10B), so dass die Schlauchleitung der Kanüle an dem Pad 22 befestigt ist. Sollte die Schlauchleitung auf Zug beansprucht werden, reißt die Perforation 38C, so dass das Pad 22 an der Punktionsstelle verbleibt.

Fig. 10C zeigt eine alternative Ausführungsform des Pad 22, das sich von der Ausführungsform von den Fig. 10A und 10B dadurch unterscheidet, dass die Schwächungslinie 38C nicht geradlinig verläuft, sondern V-förmig ausgebildet ist. In Abhängigkeit von der Länge und Form der Perforation bzw. der Größe der Fläche der Verbindungsstelle zwischen Pflaster und Pad können unterschiedliche Zugkräfte eingestellt werden, mit denen die Schlauchbefestigung gelöst werden kann. Dabei sollte die Schwächungslinie 38 derart ausgebildet sein, dass bei einer Zugbeanspruchung in Längsrichtung der Schlauchleitung eine relativ hohe Zugkraft notwendig ist, um Pflaster und Pad auseinanderreißen zu können, während bei einer Zugbeanspruchung quer zur Längsichtung sich beide Teile leicht voneinander trennen lassen. Dadurch ist es möglich, nach der Blutbehandlung das Pflaster 38 zusammen mit der Schlauchleitung 6, 7 und der Kanüle 5, 8 leicht von dem Pad 22 abzutrennen.

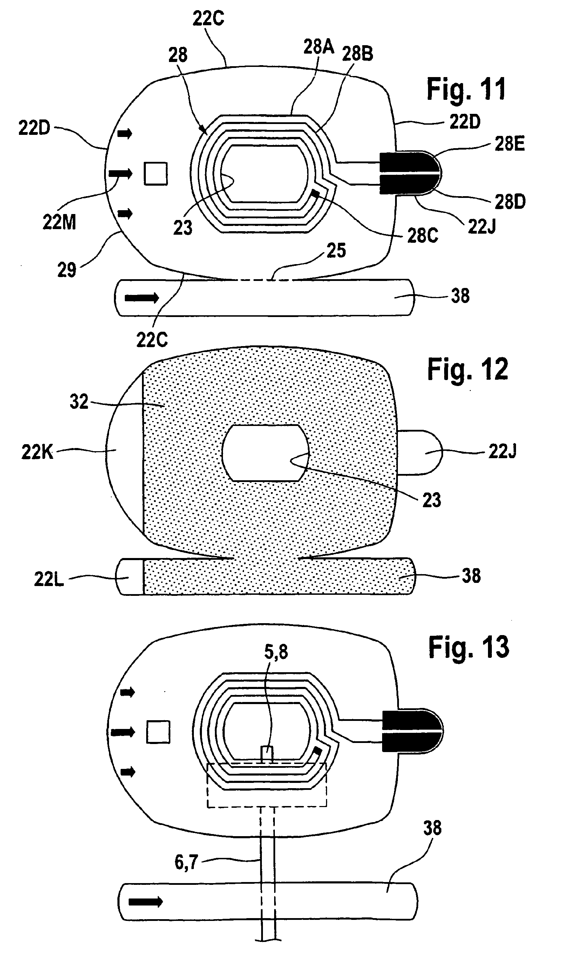

Die Figuren 11 bis 13 zeigen eine weitere Ausführmgsform des Pad 22, das sich von dem unter Bezugnahme auf die Figuren 2 bis 5 beschriebenen Pad 22 zwar durch die Form unterscheidet, aber den gleichen Schichtaufbau hat. Daher werden für die einander entsprechenden Teile auch die gleichen Bezugszeichen verwendet.

Das Pad weist eine im Wesentlichen ovale Form mit Längs- und Schmalseiten 22C, 22D auf, wobei an einer der Längsseiten 22C eine Sicherungseinrichtung 38 für die Kanüle bzw. deren Schlauchleitung angeformt ist. Die Sicherungseinrichtung ist ein schmaler Pflaster-Streifen 38, der an der Längsseite über eine Perforationslinie 25 angeschlossen ist, so dass sich der Streifen leicht von dem Pad 22 abtrennen lässt.

Der Feuchtigkeitssensor 28 und die Ausnehmung 23 befinden sich im Zentrum des ovalen Teilabschnitts des Pad 22. Damit hat das Pad einen symmetrischen Aufbau, so dass es sowohl an dem linken als auch rechten Arm des Patienten zusammen mit der arteriellen bzw. venösen Kanüle 5, 8 befestigt werden kann. Eine Perforation weist das Pad im Bereich der Ausnehmung 23 nicht auf, da das Pad dazu bestimmt ist, erst nach dem Legen der Kanüle 5, 8 auf die Haut des Patienten aufgelegt zu werden. Das wie ein Pflaster zu handhabende Pad dient nicht nur zur Detektion von Blut, sondern gleichsam zur Fixierung der Kanüle.

Das Pad 22 weist wieder ein Trägermaterial 29, insbesondere einen Vlies auf, der an seiner Unterseite mit einer für Flüssigkeit undurchlässigen, aber für Dampf durchlässigen Klebeschicht 32 versehen ist. An der Oberseite ist das Trägermaterial mit einer ersten und zweiten Leiterbahn 28A, 28B versehen, deren eine Enden an einem Abschlusswiderstand 28C angeschlossen sind und deren andere Enden an Kontaktelementen 28D, 28E angeschlossen sind. Die Leiterbahnen 28A, 28B umschließen die Ausnehmung 23 als geschlossene Leiterschleife, da eine Perforation nicht vorhanden ist. Die Klebeschicht 32 ist wieder mit einem in den Figuren nicht dargestellten Silikonpapier abgedeckt, das vor dem Gebrauch des Pad abgezogen wird.

Die nebeneinander liegenden Kontaktelemente 28D, 28E befinden sich an einer Lasche 22J, die an einer der Schmalseiten 22D angeformt ist. Die Kontaktelemente 28D, 28E an der Lasche 22J sind für einen Anschlussteil 37 mit stiftförmigen Kontakten 37A, 37B bestimmt, der unter Bezugnahme auf die Figuren 9A, 9B beschrieben ist.

Die Lasche 22J für die Kontaktelemente 28D, 28E ist frei von der Klebeschicht 32 an der Rückseite des Trägermaterials 29. Auch die der Lasche 22J für die Kontaktelemente 28D, 28E gegenüberliegenden Randbereiche 22K, 22L des Pad 22 bzw. des Pflaster-Streifens 36 sind frei von der Klebeschicht, so dass diese Bereiche Laschen bilden, an denen sie sich leicht wieder von der Haut des Patienten abziehen lassen. Aufgedruckte Pfeile 22M an den Teilabschnitten 22K, 22L des Pad 2 kennzeichnen die Zugrichtung.

Das Pad wird wie folgt gehandhabt. Zunächst wird die Kanüle 5, 8 gelegt. Dann wird der Pflaster-Streifen 36 von dem Pad 22 abgerissen und das in den Figuren nicht dargestellte Silikonpapier von dem Streifen 36 abgezogen, so dass die Klebschicht 32 freiliegt. Dann wird der Schlauch 6, 7 der Kanüle 5, 8 am Oberarm des Patienten mit dem Pflaster-Streifen 36 befestigt. Anschließend wird nach Entfernen des Silkonpapiers das Pad 22 auf die Haut des Patienten so aufgeklebt, dass die Lasche 22J mit den Kontaktelementen 28D, 28E zur Außenseite des Arms zeigt. Dadurch wird erreicht, dass sich das Verbindungskabel 44 des an das Pad anzuschließenden Verbindungsteils 37 nicht mit den arteriellen und venösen Schlauchleitungen 6, 7 verwickeln kann. Da das Pad nicht vollständig an der Unterseite mit Klebstoff bedeckt ist, lässt sich das Papier leicht an den Laschen greifen, die mit den Pfeilen 22M gekennzeichnet sind. Nunmehr wird das Anschlussteil 37 mit den stiftförmigen Kontakten 37A, 37B an den Kontaktelementen 28D, 28E der Lasche 22J angeschlossen, so dass die Vorrichtung in Betrieb genommen werden kann.

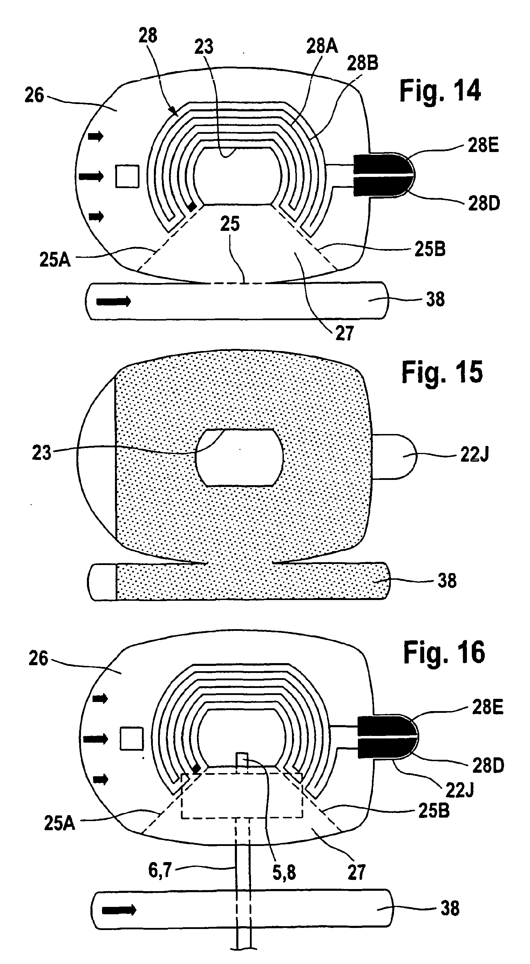

Die Figuren 14 bis 16 zeigen eine alternative Ausführungsform des Pad 22, das sich von dem unter Bezugnahme auf die Figuren 11 bis 13 beschriebenen Pad 22 nur in der Ausbildung des Feuchtigkeitssensors 28 und der Unterteilung des Pad in einen ersten und zweiten Teilabschnitt 26 und 27 unterscheidet. Die einander entsprechenden Teile werden daher wieder mit den gleichen Bezugszeichen bezeichnet. Die beiden Teilabschnitte 26 und 27 werden von zwei schräg aufeinander zu laufenden Sollbruchlinien 25A, 25B von einander getrennt, die sich jeweils von dem Rand einer Längsseite 22C bis zu der Ausnehmung 23 des Pad 22 erstrecken. Dadurch wird ein erster Teilabschnitt 26 geschaffen, auf dem sich der Feuchtigkeitssensor 28 befindet, und ein zweiter im Wesentlichen trapezförmiger Teilabschnitt 27, unter dem sich die Kanüle 5, 8 bzw. die Schlauchleitung 6, 7 erstreckt, wenn das Pad auf die Haut des Patienten geklebt ist (Fig. 16). Die beiden Leiterbahnen 28A, 28B des Feuchtigkeitssensors 28 erstrecken sich bis an die Sollbruchlinien 25A, 25B, so dass die Leiterschleife die Ausnehmung 23 nicht vollständig umschließt.

Die alternative Ausführungsform des Pad von den Figuren 14 bis 16 wird wie die Ausführungsform von den Figuren 11 bis 13 gehandhabt. Das Pad wird nach dem Legen der Kanüle 5, 8 auf die Haut des Patienten geklebt, wobei die Kanüle bzw. die Schlauchleitung unter dem Pad liegt. Mit dem Pflaster-Streifen 36 wird die Schlauchleitung zusätzlich fixiert. Die alternative Ausführungsform hat den Vorteil, dass bei einem Zug an der Kanüle das Pad nicht vollständig von der Haut des Patienten abgerissen wird, sondern lediglich der Teilabschnitt 27 von dem Pad 22 abgetrennt wird, an dem die Kanüle befestigt ist, wobei die beiden Sollbruchlinien 25A, 25B bis zu der Ausnehmung 23 einreißen. Dabei werden die Leiterbahnen 28A, 28B des Feuchtigkeitssensors 28 aber nicht durchtrennt. Die Vorrichtung bleibt also auch nach dem Abreißen der Nadel voll funktionsfähig, so dass der Austritt von Blut an der Punktionsstelle erkannt werden kann.

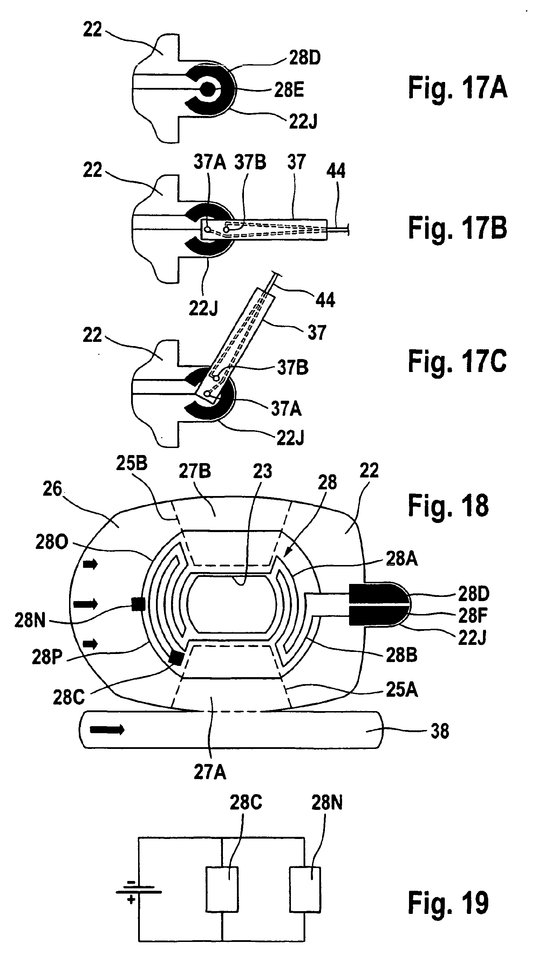

Die Figuren 17A bis 17C zeigen eine alternative Ausführungsform der Kontaktelemente 28D, 28E, die an der Lasche 22J des Pad von den Figuren 14 bis 16 oder von den Figuren 11 bis 13 vorgesehen sein können. Das erste Kontaktelement 28E ist kreisförmig und im Zentrum der Lasche 22J angeordnet, während das zweite Kontaktelement 28D kreisbogenförmig ausgebildet ist und das erste Kontaktelement 28E umschließt. An die Kontaktelemente 28D, 28E wird der Anschlussteil 37 mit den stiftförmigen Kontakten 37A, 37B angeschlossen, der unter Bezugnahme auf die Figuren 9A, 9B beschrieben ist. Der Vorteil der alternativen Ausführungsform der Kontaktelemente liegt darin, dass der Anschlussteil 37 nicht nur von einer Seite, sondern aus verschiedenen Richtungen an das Pad 22 angeschlossen werden kann. Die Figuren 17B und 17C zeigen den aus zwei verschiedenen Richtungen an das Pad angeschlossenen Anschlussteil 37. Das Verbindungskabel 44 kann somit so verlaufen, wie es die jeweilige Anschlusssituation erfordert.

Die Figuren 18 und 19 zeigen eine weitere alternative Ausführungsform des Pad 22, die sich von dem unter Bezugnahme auf die Figuren 14 bis 16 beschriebenen Pad 22 in der Ausbildung des Feuchtigkeitssensors 28 unterscheidet. Ein weiterer Unterschied liegt darin, dass nicht ein abtrennbarer Teilabschnitt, sondern zwei abtrennbare im Wesentlichen trapezförmige Teilabschnitte 27A, 27B vorgesehen sind, die symmetrisch auf beiden Seiten der Ausnehmung 23 des Pad 22 angeordnet sind. Die einander entsprechenden Teile werden wieder mit den gleichen Bezugszeichen bezeichnet.

Die abtrennbaren Teilabschnitte 27A, 27B werden von einer ersten und zweiten Sollbruchlinie 25A, 25B von dem anderen Teil des Pad 22 getrennt. Die Sollbruchlinien 25A, 25B weisen jeweils einen Abschnitt, der von dem Rand einer Längsseite 22D des Pad bis nahe an die Ausnehmung 23 verläuft, einen im Abstand zu der Ausnehmung 23 verlaufenden mittleren Abschnitt und einen bis zu dem Rand der Längsseite des Pad verlaufenden dritten Abschnitt auf. Aufgrund des symmetrischen Aufbaus kann das Pad in Abhängigkeit von der jeweiligen Anschlusssituation am linken oder rechten Arm des Patienten befestigt werden. Dabei wird die Kanüle 5, 8 mit der Schlauchleitung 6, 7 unter einem der beiden abtrennbaren Teilabschnitte 27A, 27B geführt.

Der Feuchtigkeitssensor 28 weist eine zu einem ersten Abschlusswiderstand 28C führende erste Leiterbahn 28A und eine von dem Abschlusswiderstand abgehende zweite Leiterbahn 28B auf, wobei die Enden der ersten und zweiten Leiterbahn mit den Kontaktelementen 28E, 28D elektrisch verbunden sind, die auf die Lasche 22J aufgebracht sind. Beide Leiterbahnen 28A, 28B bilden eine die Ausnehmung 23 des Pad 22 umschließende Leiterschleife, die sich nicht über die beiden abtrennbaren Teilabschnitte 27A, 27B des Pad erstreckt

Dem ersten Abschlusswiderstand 28C ist ein zweiter Abschlusswiderstand 28N parallel geschaltet. Fig. 19 zeigt das elektrische Ersatzschaltbild mit der Parallelschaltung beider Abschlusswiderstände 28C, 28N. Von der ersten und zweiten Leiterbahn 28A, 28B geht eine dritte und vierte Leiterbahn 28O, 28P ab, die zu dem zweiten Abschlusswiderstand 28N führen. Im Gegensatz zu der ersten und zweiten Leiterbahn 28A, 28B verlaufen die dritte und vierte Leiterbahn 28O, 28P jeweils durch einen der beiden abtrennbaren Teilabschnitte 27A, 27B des Pad 22.

Wenn einer der beiden Teilabschnitte 27A, 27B bei einem Zug an der Kanüle oder der Schlauchleitung abgerissen werden sollte, bleiben die erste und zweite Leiterbahn 28A, 28B unversehrt, da diese Leiterbahnen die Sollbruchlinien 25A, 25B nicht kreuzen, die dritte bzw. vierte Leiterbahn 280 bzw. 28P hingegen wird durchtrennt, da diese Leiterbahn durch den abgetrennten Abschnitt 27A bzw. 27B verläuft.

Wenn also ein Teilabschnitt 27A bzw. 27B abgerissen werden sollte, ändert sich der Widerstand des Netzwerks. Das Netzwerk hat dann nur einen Widerstand, der dem ersten Widerstand 28C entspricht. Ansonsten hat das Netzwerk, einen Widerstand, der durch die Parallelschaltung der Widerstände 28C und 28N bestimmt ist. Für den Fall einer Blutung kann an den Kontaktelementen 28E, 28D ein Widerstand gemessen werden, der von dem Widerstand abweicht, der der durch die Parallelschaltung der Widerstände 28C und 28N bestimmt ist. Die Auswerteinheit 41 überwacht den Widerstand zwischen den Kontaktelementen und stellt fest, ob eine Blutung vorliegt oder die Kanüle zusammen mit dem jeweiligen Teilabschnitt 27A, 27B des Pad 22 abgerissen ist. Auf eine Blutung wird dann geschlossen, wenn der an den Kontaktelementen gemessene Widerstand um einen vorgegebenen Betrag von dem Widerstand abweicht, der sich aus der Parallelschaltung beider Widerstände ergibt. Selbst wenn die Kanüle mit dem Teilabschnitt abgerissen ist, bleibt die Vorrichtung funktionsfähig. In diesem Fall wird auf eine Blutung geschlossen, wenn der gemessene Widerstand um einen vorgegebenen Betrag von dem ersten Widerstand 28C abweicht.

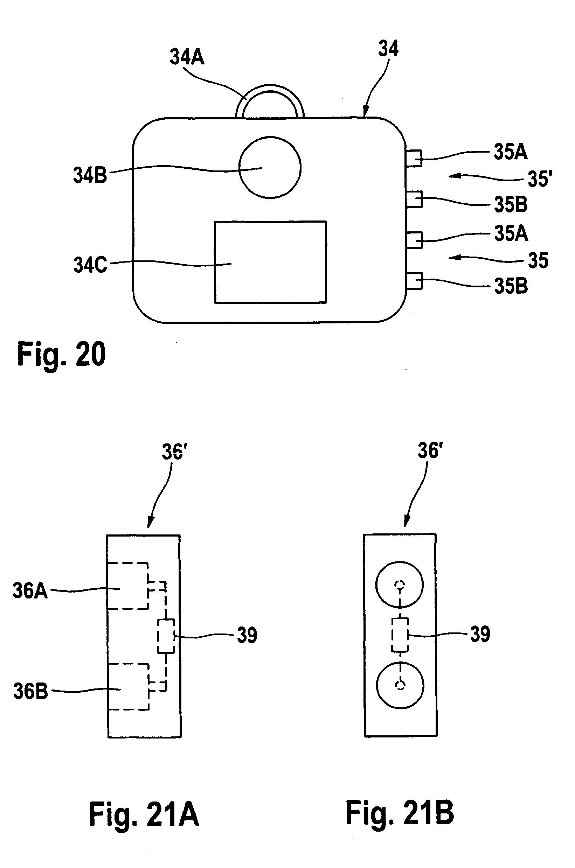

Fig. 20 zeigt ein weiteres Ausführungsbeispiel des Gehäuses der Vorrichtung zur Überwachung B des arteriellen und venösen Gefäßzugangs, das sich von der unter Bezugnahme auf Fig. 6 beschriebenen Ausführungsform dadurch unterscheidet, dass anstelle von einer Stecker-Einheit 35 mit zwei nebeneinander liegenden Steckern 35A, 35B zwei Stecker-Einheiten 35 und 35' mit jeweils zwei Steckern 35A, 35B vorgesehen sind. Die einander entsprechenden Teile sind mit den gleichen Bezugszeichen versehen. Die zusätzliche Stecker-Einheit 35' erlaubt den Anschluss einer weiteren Detektionsvorrichtung 40 an die Auswerteinheit 41 der Überwachungsvorrichtung B mit der in Fig. 7A und Fig. 7B gezeigten Buchsen-Einheit 36.

Für den Fall, dass nur eine Detektionsvorrichtung 40 an die Auswerteinheit 41 angeschlossen werden soll, kann die Stecker-Einheit, die nicht mit einer Buchsen-Einheit verbunden ist, mit einer Schutzkappe verschlossen werden, die einen isolierenden Schutz für die Kontakte bildet. Die Figuren 21A und 21B zeigen ein Ausführungsbeispiel der Schutzkappe 36', die wie die Buchsen-Einheit 36 der Figuren 7A und 7B beschaffen ist. Die beiden Buchsen 36A, 36B der Schutzkappe 36' sind mit einem Widerstand 39 verbunden, dessen elektrischer Widerstand dem elektrischen Widerstand einer intakten Detektionsvorrichtung 40 entspricht. Es ist aber auch möglich, einen derartigen Abschlusswiderstand in der Schutzkappe nicht vorzusehen.

Die Auswerteinheit 41 kann derart ausgebildet sein, dass vor der Durchführung der Behandlung der elektrische Widerstand zwischen den Steckern bzw. Buchsen der SteckerBuchsen-Einheit 35, 36 gemessen wird und der gemessene Widerstand mit einem Sollwert für den Widerstand verglichen wird, der dem Abschlusswiderstand einer intakten Detektionsvorrichtung 40 entspricht. Wenn der gemessene Wert für den Widerstand um einen vorgegebenen Betrag von dem Sollwert abweicht, schließt die Auswerteinheit 41 darauf, dass die Detektionsvorrichtung 40 defekt ist.

Eine bevorzugte Ausführungsform sieht vor, dass der Benutzer zu Beginn der Behandlung vorgeben kann, ob die Überwachung mit einer oder zwei Detektionsvorrichtungen erfolgen soll. Bei einer ersten Ausführungsform verfügt die Überwachungsvorrichtung B über eine Eingabeeinheit 41 A, auf der eingegeben werden kann, ob eine oder zwei Detektionsvorrichtungen eingesetzt werden sollen. Der Benutzer kann hierzu von einer nicht dargestellten Signaleinheit aufgefordert werden. Die Auswerteinheit kann auch derart ausgebildet sein, dass nach der Eingabe überprüft wird, ob tatsächlich ein oder zwei Detektionsvorrichtungen angeschlossen sind. Bei einer alternativen Ausführungsform wird die nicht dargestellte Eingabeeinheit der Blutbehandlungsvorrichtung dazu verwendet, einzugeben, ob ein oder zwei Detektionsvorrichtungen eingesetzt werden.

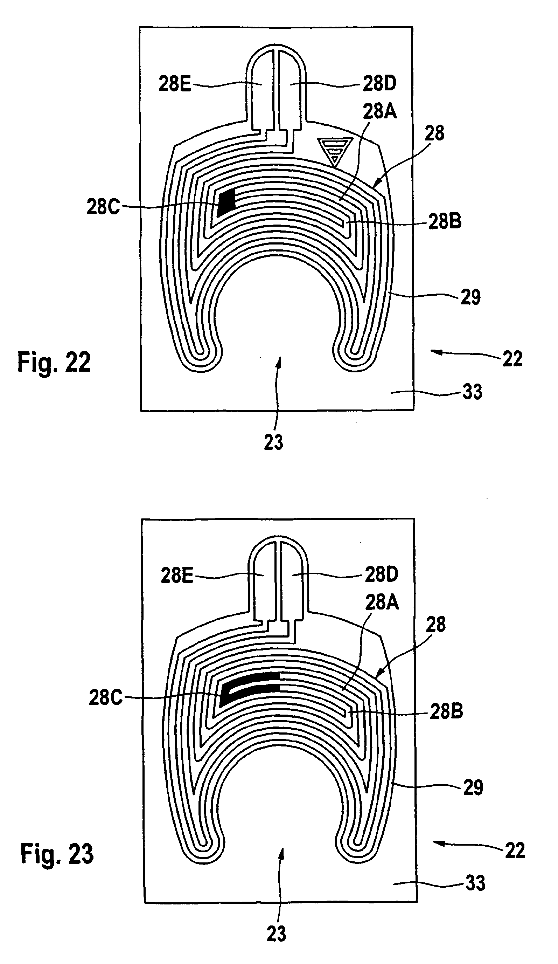

Fig. 22 zeigt ein weiteres Ausführungsbeispiel der Vorrichtung zum Detektieren von Flüssigkeit, die sich von den zuvor beschriebenen Ausführungsformen durch das Layout unterscheidet, wobei die einander entsprechenden Teile wieder mit den gleichen Bezugszeichen versehen sind. Der Feuchtigkeitssensor 28 weist wieder zwei Leiterbahnen 28A, 28B auf, die von den Kontaktelementen 28D und 28E zu einem Abschlusswiderstand 28C führen, der wieder auf das Trägermaterial aufgedruckt ist. Das Ausführungsbeispiel von Fig. 22 zeichnet sich dadurch aus, dass die Ausnehmung 23 für die Punktionsnadel nicht im Zentrum des Pad 22 liegt, so dass die Ausnehmung von dem Pad nicht vollständig umschlossen wird. Das die Ausnehmung nur über einen Teil des Umfangs umschließende Pad 22 ermöglicht ein besonders leichtes Anbringen des Pad an einen bereits punktierten Patientenzugang. Auf eine Fixierungshilfe zur Fixierung der Nadel wird bei dieser Ausführungsform verzichtet.

Bei dem Ausführungsbeispiel von Fig. 22 ist das Trägermaterial 29 des Pad 22 mit einer Klebeschicht 32 auf ein Silikonpapier 33 aufgebracht, das größer als das Trägermaterial des Pad 22 ist (Fig. 4). Das Silikonpapier 33 ist bei diesem Ausführungsbeispiel ein rechteckförmiges Kaschierpapier, das an allen Seiten über den äußeren Rand des Trägermaterials hinausragt. Durch Biegen an den überstehenden Rändern kann das Trägermaterial 29 leicht von dem Silikonpapier 33 abgezogen werden. Das Abziehen des Trägermaterials wird auch dadurch erleichtert, dass die Fläche, auf der die Kontaktelemente 28D, 28E aufgebracht sind, als Lasche ausgebildet ist, die der Ausnehmung vorzugsweise gegenüberliegt, aber auch an einer anderen Seite vorgesehen sein kann. Es können aber auch eine oder mehrere Abziehlaschen ohne Kontaktelemente vorgesehen sein.

Anstelle eines Silikonpapiers können auch so genannte Liner aus anderen Materialien verwendet werden, von denen sich das Trägermaterial leicht abziehen lässt.

Fig. 23 zeigt ein Ausführungsbeispiel der Detektionsvorrichtung 40, die sich von der Ausführungsfonn von Fig. 22 nur dadurch unterscheidet, dass der Abschlusswiderstand 28C als eine langgezogene flächenhafte Struktur aufgebracht ist, wodurch eine kleinere fertigungsbedingte Streuung des elektrischen Widerstands erzielt wird. Diese langgezogene flächenhafte Struktur kann als Abschnitt der aufgedruckten Leiterbahn ausgebildet sein. Die Breite dieser Struktur kann der Breite der Leiterbahn entsprechen.

## Patentansprüche

1. Vorrichtung zum Detektieren von Feuchtigkeit zur Verwendung bei einer Vorrichtung zur Überwachung eines Zugangs zu einem Patienten für eine Einrichtung, mit der über eine Schlauchleitung eine Flüssigkeit zu einem Patienten geführt und/oder von dem Patienten abgeführt wird, insbesondere zur Überwachung des Gefäßzugangs bei einer extrakorporalen Blutbehandlung, bei der Blut eines Patienten über eine arterielle Schlauchleitung, die eine arterielle Kanüle aufweist, von dem Patienten abgeführt wird, und über eine venöse Schlauchleitung, die eine venöse Punktionskanüle aufweist, dem Patienten zugeführt wird, wobei
die Vorrichtung zum Detektieren von Feuchtigkeit als eine auf die Haut des Patienten aufzulegende Abdeckung (22) aus einem flexiblen Material ausgebildet ist, die eine der Haut des Patienten abgewandte Oberseite (22A) und eine der Haut des Patienten zugewandte Unterseite (22B) aufweist, und
die eine Ausnehmung (23) zum Durchführen einer Kanüle für den Patientenzugang und einen Feuchtigkeitssensor (28) mit Kontaktelementen (28E, 28D; 28G, 28F) zum Anschluss des Feuchtigkeitssensors aufweist,
**dadurch gekennzeichnet, dass**
die Abdeckung (22) an der Unterseite (22B) für Flüssigkeit nicht durchlässig ist.

2. Vorrichtung zum Detektieren von Feuchtigkeit nach Anspruch 1, **dadurch gekennzeichnet, dass** die Abdeckung (22) an der Unterseite (22B) für Luft und Wasserdampf durchlässig ist.

3. Vorrichtung zum Detektieren von Feuchtigkeit nach Anspruch 1 oder 2, **dadurch gekennzeichnet, dass** die Abdeckung (22) an der Oberseite (22A) für Flüssigkeit durchlässig ist.

4. Vorrichtung nach einem der Ansprüche 1 bis 3, **dadurch gekennzeichnet, dass** die Ausnehmung (23) in der Abdeckung (22) eine im Wesentlichen kreisförmige oder ovale Ausnehmung ist.

5. Vorrichtung nach einem der Ansprüche 1 bis 4, **dadurch gekennzeichnet, dass** die Abdeckung (22) ein flexibles Trägermaterial (29) aufweist, wobei auf die Oberseite des Trägermaterials mindestens eine Leiterbahn (28A, 28B) aus einem flexiblen Material und mindestens zwei Kontaktelemente (28E, 28D; 28G, 28F) zum Anschluss der mindestens einen Leiterbahn aufgebracht sind.

6. Vorrichtung nach Anspruch 5, **dadurch gekennzeichnet, dass** das Trägermaterial (29) ein Vlies ist.

7. Vorrichtung nach Anspruch 5 oder 6, **dadurch gekennzeichnet, dass** die mindestens eine Leiterbahn (28A, 28B) eine auf das Trägermaterial (29) aufgedruckte Leiterbahn ist.

8. Vorrichtung nach einem der Ansprüche 5 bis 7, **dadurch gekennzeichnet, dass** eine zu einem Abschlusswiderstand (28C) führende erste Leiterbahn (28A) und eine von dem Abschlusswiderstand abgehende zweite Leiterbahn (28B) vorgesehen ist, wobei die Enden der ersten und zweiten Leiterbahn mit den Kontaktelementen (28E, 28D; 28G, 28F) elektrisch verbunden sind, die auf das Trägermaterial (29) aufgebracht sind, und der erste oder zweite Abschlusswiderstand (28C, 28N) ein auf das Trägermaterial (29) aufgedruckter Abschlusswiderstand ist.

9. Vorrichtung nach einem der Ansprüche 5 bis 8, **dadurch gekennzeichnet, dass** die mindestens eine Leiterbahn (28A, 28B) mindestens eine die Ausnehmung (23) in der Abdeckung zumindest teilweise umschließende Leiterschleife bildet.

10. Vorrichtung nach einem der Ansprüche 1 bis 9, **dadurch gekennzeichnet, dass** die Abdeckung (22) an der Unterseite (22B) eine Klebeschicht (32) aufweist, so dass die Abdeckung an der Haut des Patienten haftend befestigbar ist.

11. Vorrichtung nach Anspruch 10, **dadurch gekennzeichnet, dass** die Klebeschicht (32) eine für Flüssigkeit undurchlässige Klebeschicht ist, die sich nur über einen Teilabschnitt (26) der Unterseite (22B) der Abdeckung (22) erstreckt, so dass ein anderer Teilabschnitt (27) der Abdeckung an der Unterseite frei von der Klebeschicht ist, wobei der von der Klebeschicht freie Teilabschnitt (27) der Abdeckung (22) der Bereich der Abdeckung ist, an dem die Kontaktelemente (28E, 28D; 28G, 28F) für die mindestens eine Leiterbahn (28A, 28B) angeordnet sind.

12. Vorrichtung zur Überwachung eines Zugangs zu einem Patienten für eine Einrichtung, mit der über eine Schlauchleitung eine Flüssigkeit zu dem Patienten geführt oder von dem Patienten abgeführt wird, insbesondere zur Überwachung des Gefäßzugangs mit einer extrakorporalen Blutbehandlungsvorrichtung, bei der Blut eines Patienten über eine arterielle Schlauchleitung, die eine arterielle Kanüle aufweist, von dem Patienten abgeführt wird, und über eine venöse Schlauchleitung, die eine venöse Punktionskanüle aufweist, dem Patienten zugeführt wird, und mit einer Vorrichtung zum Detektieren von Feuchtigkeit nach einem der Ansprüche 1 bis 11.

13. Vorrichtung zur Überwachung eines Zugangs zu einem Patienten nach Anspruch 12, **dadurch gekennzeichnet, dass** die Überwachungsvorrichtung (B) eine an den Feuchtigkeitsensor (28) der Vorrichtung zum Detektieren von Feuchtigkeit (40) anschließbare Auswerteinheit (41) aufweist, dass die Überwachungsvorrichtung (B) ein Verbindungskabel (44) zur Herstellung einer Verbindung zwischen dem Feuchtigkeitssensor (28) und der Auswerteinheit (41) aufweist, wobei das Verbindungskabel (44) einen Anschlussteil (37) mit federnden Kontakten (37A, 37B) aufweist, die mit den Kontaktelementen (28E, 28D; 28G, 28F) des Feuchtigkeitssensors elektrisch verbindbar sind, und dass die Auswerteinheit (41) eine Einheit zum Bestimmen des Widerstandes oder der Kapazität zwischen den Kontaktelementen (28E, 28D; 28G, 28F) des Feuchtigkeitssensors (28) aufweist.

14. Blutbehandlungsvorrichtung mit einem extrakorporalen Blutkreislauf, der eine arterielle Schlauchleitung (6) mit einer arteriellen Kanüle (5) und eine venöse Schlauchleitung (7) mit einer venösen Kanüle (8) aufweist, und mit einer Vorrichtung (B) zur Überwachung des arteriellen und/oder venösen Gefäßzugangs nach Anspruch 12 oder 13.

15. Blutbehandlungsvorrichtung nach Anspruch 14, **dadurch gekennzeichnet, dass** die Blutbehandlungsvorrichtung eine Steuereinheit (15) aufweist, wobei die Überwachungsvorrichtung (B) mit der Steuereinheit (15) der Blutbehandlungsvorrichtung derart zusammenwirkt, dass ein Steuersignal zur Auslösung eines Alarms und/oder eines Eingriffs in die Maschinensteuerung erzeugt wird, wenn Feuchtigkeit mit der Vorrichtung zum Detektieren von Feuchtigkeit detektiert wird.

## Claims

1. Device for detecting moisture for use in a device for monitoring an access to a patient for an apparatus with which a fluid can be supplied to a patient and/or carried away from the patient via a hose line, in particular for monitoring the vascular access in an extracorporeal blood treatment, in which blood of a patient is carried away from the patient via an arterial hose line which has an arterial cannula, and is supplied back to the patient via a venous hose line which has a venous puncture cannula, the device for detecting moisture being configured as a cover (22) which is made of a flexible material and is to be placed onto the skin of the patient, said cover having an upper surface (22A) facing away from the skin of the patient and a lower surface (22B) facing the skin of the patient, and a recess (23) for allowing passage therethrough of a cannula for access to the patient and a moisture sensor (28) comprising contact elements (28E, 28D; 28G, 28F) for connecting the moisture sensor, **characterised in that** the cover (22) is impermeable to fluid on the lower surface (22B).

2. Device for detecting moisture according to claim 1, **characterised in that** the cover (22) is permeable to air and water vapour on the lower surface (22B).

3. Device for detecting moisture according to either claim 1 or claim 2, **characterised in that** the cover (22) is permeable to fluid on the upper surface (22A).

4. Device according to any of claims 1 to 3, **characterised in that** the recess (23) in the cover (22) is a substantially circular or oval recess.

5. Device according to any of claims 1 to 4, **characterised in that** the cover (22) comprises a flexible support material (29), at least one strip conductor (28A, 28B) made of a flexible material and at least two contact elements (28E, 28D; 28G, 28F) for connecting the at least one strip conductor being applied to the upper surface of the support material.

6. Device according to claim 5, **characterised in that** the support material (29) is a nonwoven fabric.

7. Device according to either claim 5 or claim 6, **characterised in that** the at least one strip conductor (28A, 28B) is printed on the support material (29).

8. Device according to any of claims 5 to 7, **characterised in that** a first strip conductor (28A) leading to a terminating resistor (28C) and a second strip conductor (28B) leading away from the terminating resistor are provided, the ends of the first and second strip conductor being electrically connected to the contact elements (28E, 28D; 28G, 28F) which are applied to the support material (29) and the first or second terminating resistor (28C, 28N) being printed on the support material (29).

9. Device according to any of claims 5 to 8, **characterised in that** the at least one strip conductor (28A, 28B) forms at least one conductor loop which surrounds the recess (23) in the cover at least in part.

10. Device according to any of claims 1 to 9, **characterised in that** the cover (22) comprises an adhesive layer (32) on the lower surface (22B), so that the cover can be adhesively fixed to the skin of the patient.

11. Device according to claim 10, **characterised in that** the adhesive layer (32) is impermeable to fluid and extends only over a portion (26) of the lower surface (22B) of the cover (22), so that another portion (27) of the lower surface of the cover is free from the adhesive layer, the portion (27) of the cover (22) which is free from the adhesive layer being the region of the cover on which the contact elements (28E, 28D; 28G, 28F) for the at least one strip conductor (28A, 28B) are arranged.

12. Device for monitoring an access to a patient for an apparatus with which a fluid is supplied to the patient or carried away from the patient via a hose line, in particular for monitoring the vascular access with an extracorporeal blood treatment apparatus in which blood of a patient is carried away from the patient via an arterial hose line which has an arterial cannula and is supplied back to the patient via a venous hose line which has a venous puncture cannula, said device comprising a device for detecting moisture according to any of claims 1 to 11.

13. Device for monitoring an access to a patient according to claim 12, **characterised in that** the monitoring device (B) comprises an evaluation unit (41) which can be connected to the moisture sensor (28) of the device for detecting moisture (40), **in that** the monitoring device (B) comprises a connection cable (44) for producing a connection between the moisture sensor (28) and the evaluation unit (41), the connection cable (44) comprising a connection part (37) having resilient contacts (37A, 37B) which can be electrically connected to the contact elements (28E, 28D; 28G, 28F) of the moisture sensor, and **in that** the evaluation unit (41) comprises a unit for determining the resistance or the capacitance between the contact elements (28E, 28D; 28G, 28F) of the moisture sensor (28).

14. Blood treatment device having an extracorporeal blood circuit, the device comprising an arterial hose line (6) having an arterial cannula (5) and a venous hose line (7) having a venous cannula (8), and comprising a device (B) for monitoring the arterial and/or venous vascular access according to either claim 12 or claim 13.

15. Blood treatment device according to claim 14, **characterised in that** the blood treatment device comprises a control unit (15), the monitoring device (B) cooperating with the control unit (15) of the blood treatment device in such a way that a control signal for triggering an alarm and/or an intervention in the machine control is generated if moisture is detected by the device for detecting moisture.

## Revendications

1. Dispositif de détection de l'humidité, pour utilisation dans un dispositif de surveillance d'un accès à un patient pour un dispositif à l'aide duquel un liquide est guidé vers un patient par l'intermédiaire d'une ligne flexible et/ou est évacué du patient, en particulier pour la surveillance de l'accès vasculaire lors d'un traitement extracorporel du sang, au cours duquel du sang provenant d'un patient est, par une ligne flexible artérielle qui comprend une canule artérielle, évacué du patient, et par l'intermédiaire d'une ligne flexible veineuse qui présente une canule de ponction veineuse est évacué du patient,
le dispositif de détection de l'humidité étant configuré comme un timbre (22), destiné à être placé sur la peau du patient, en un matériau flexible, et qui présente une face supérieure (22A) opposée à la peau du patient et une face inférieure (22B) en regard de la peau du patient, et
qui comprend un évidement (23) pour le passage d'une canule, pour permettre un accès au patient, et un capteur d'humidité (28) comportant des éléments de contact (28E, 28D ; 28G, 28F) pour raccordement au capteur d'humidité,
**caractérisé en ce que** le timbre (22) est, sur sa face inférieure (22B), imperméable aux liquides.

2. Dispositif de détection de l'humidité selon la revendication 1, **caractérisé en ce que** le timbre (22) est, sur sa face inférieure (22B), perméable à l'air et à la vapeur d'eau.

3. Dispositif de détection de l'humidité selon la revendication 1 ou 2, **caractérisé en ce que** le timbre (22) est, sur sa face supérieure (22A), perméable aux liquides.

4. Dispositif selon l'une des revendications 1 à 3, **caractérisé en ce que** l'évidement (23) aménagé dans le timbre (22) est un évidement pour l'essentiel circulaire ou ovale.

5. Dispositif selon l'une des revendications 1 à 4, **caractérisé en ce que** le timbre (22) comprend un matériau support flexible (29), une piste conductrice (28A, 28B), constituée d'un matériau flexible, et au moins deux éléments de contact (28E, 28D ; 28G, 28F), pour le raccordement de la ou des pistes conductrices, étant appliqués sur la face supérieure du matériau support.

6. Dispositif selon la revendication 5, **caractérisé en ce que** le matériau support (29) est un non-tissé.

7. Dispositif selon la revendication 5 ou 6, **caractérisé en ce que** la ou les pistes conductrices (28A, 28B) sont des pistes conductrices imprimées sur le matériau support (29).

8. Dispositif selon l'une des revendications 5 à 7, **caractérisé en ce que** sont prévues une première piste conductrice (28A) allant vers une résistance de terminaison (28C) et une deuxième piste conductrice (28B) partant de la résistance de terminaison, les extrémités de la première et de la deuxième pistes conductrices étant électriquement reliées aux éléments de contact (28E, 28D ; 28G, 28F), qui sont appliqués sur le matériau support (29), et la première ou la deuxième résistance de terminaison (28C, 28N) est une résistance de terminaison imprimée sur le matériau support (29).

9. Dispositif selon l'une des revendications 5 à 8, **caractérisé en ce que** la ou les pistes conductrices (28A, 28B) forment au moins une boucle conductrice entourant au moins partiellement l'évidement (23) aménagé dans le timbre.

10. Dispositif selon l'une des revendications 1 à 9, **caractérisé en ce que** le timbre (22) comprend sur sa face inférieure (22B) une couche adhésive (32), de telle sorte que le timbre puisse être fixé par adhérence à la peau du patient.

11. Dispositif selon la revendication 10, **caractérisé en ce que** la couche adhésive (32) est une couche adhésive imperméable aux liquides, qui ne s'étend que sur un segment partiel (26) de la face inférieure (22B) du timbre (22), de telle sorte qu'un autre segment partiel (27) du timbre soit, sur la face inférieure, exempt de la couche adhésive, le segment partiel (27), exempt de la couche adhésive, du timbre (22) étant la zone du timbre sur laquelle sont disposés les éléments de contact (28E, 28D ; 28G, 28F) pour la ou les pistes conductrices (28A, 28B).

12. Dispositif de surveillance d'un accès à un patient pour un dispositif à l'aide duquel un liquide est envoyé au patient ou est évacué du patient par une ligne flexible, en particulier pour surveiller l'accès vasculaire faisant appel à un dispositif extracorporel de traitement du sang, dans lequel du sang d'un patient est, par une ligne flexible artérielle qui comprend une canule artérielle, évacué du patient et, par une ligne flexible veineuse qui comprend une canule de ponction veineuse, est évacué du patient, et comportant un dispositif de détection de l'humidité selon l'une des revendications 1 à 11.

13. Dispositif de surveillance d'un accès à un patient selon la revendication 12, **caractérisé en ce que** le dispositif de surveillance (B) comprend une unité d'évaluation (41), pouvant être raccordée au capteur d'humidité (28) du dispositif de détection de l'humidité (40), **en ce que** le dispositif de surveillance (B) comprend un câble de liaison (44) pour réaliser une liaison entre le capteur d'humidité (28) et l'unité d'évaluation (41), le câble de liaison (44) comprenant une pièce de raccordement (37) munie de contacts élastiques (37A, 37B), qui peuvent être électriquement reliés aux éléments de contact (28E, 28D ; 28G, 28F) du capteur d'humidité, et **en ce que** l'unité d'évaluation (41) comprend une unité pour déterminer la résistance ou la capacité entre les éléments de contact (28E, 28D ; 28G, 28F) du capteur d'humidité (28).

14. Dispositif de traitement du sang comprenant une circulation extracorporelle du sang, qui comprend une ligne flexible artérielle (6) munie d'une canule artérielle (5) et une ligne flexible veineuse (7) munie d'une canule veineuse (8), et comportant un dispositif (B) pour surveiller l'accès vasculaire artériel et/ou veineux selon la revendication 12 ou 13.

15. Dispositif de traitement du sang selon la revendication 14, **caractérisé en ce que** le dispositif de traitement du sang comprend une unité de commande (15), le dispositif de surveillance (B) coopérant avec l'unité de commande (15) du dispositif de traitement du sang de façon à produire un signal de commande pour déclencher une alarme et/ou une intervention dans le système de commande de la machine quand de l'humidité est détectée à l'aide du dispositif de détection de l'humidité.
